# EUROPEAN PATENT APPLICATION

(11) **EP 4 393 946 A1**
(43) Date of publication of application: **03.07.2024**
(21) Application number: 22860655.4
(22) Date of filing: 26.08.2022
(51) Int. Cl.: C07K 16/18, A61K 39/395

(54) **CDC PLATFORM ANTIBODY**

(30) Priority: 27.08.2021 CN 202110997571
(71) Applicant: Sunshine Guojian Pharmaceutical (Shanghai) Co., Ltd., Shanghai 201203 (CN)
(72) Inventor: ZHAO, Jie, Shanghai 201203 (CN); ZHANG, Xuesai, Shanghai 201203 (CN); HUANG, Haomin, Shanghai 201203 (CN); ZHU, Zhenping, Shanghai 201203 (CN)
(74) Representative: reuteler & cie SA
(86) International application number: PCT/CN2022/115262
(87) International publication number: WO 2023/025309

(57) **Abstract**

A platform technology for enhancing a CDC effect. Specifically, the present invention relates to an antibody having antigen binding activity and CDC activity, or a fusion protein or fragment thereof; said antibody or the fusion protein or fragment thereof comprises a heavy chain Fc region element and a binding domain targeting a predetermined antigen, where said heavy chain Fc region element: (1) comprises an amino acid residue at position 309 of the Fc region that is selected from the following group: W, Y, E, F, H, C, D, N, Q, R, S, T, or K, and preferably further comprises R at position 345 of the Fc region; and/or (2) has a tail-piece element at a C-terminus. The antibody or the fusion protein or fragment thereof can significantly improve a CDC property of the antibody or fusion protein or fragment thereof of the present invention by means of site-directed mutagenesis of an amino acid at position 309 or the addition of a tail-piece element at a C-terminus, a therapeutic effect of an existing antibody or fusion protein or fragment thereof can also be improved, and a variety of diseases can be treated

## Description

### Technical field

The present invention relates to the field of antibodies, specifically to a complement dependent cytotoxicity (CDC) and/or antibody dependent cell-mediated cytotoxicity (ADCC) platform antibody.

### Background

Complement (C) is a group of activated proteins with enzymatic activity present in normal human and animal serum and tissue fluids. As early as the end of the 19th century, Bordet confirmed that fresh blood contains a heat-resistant component that can assist and supplement specific antibodies and mediate immune lysobacteriolytic and hemolytic effects, so it is called complement. The complement system is a multi-molecular system composed of over 30 soluble proteins, membrane-bound proteins, and complement receptors, hence it is called the complement system. Under normal circumstances, complement is a component of plasma proteins. The components of the complement system exist in plasma as inactive precursors. When needed, it is activated in turn under the action of an activator, such as an antigen-antibody complex, and finally plays a role. According to the biological functions of each component of the complement system, it can be divided into intrinsic complement components, regulatory complement components, and complement receptors.

The intrinsic components of complement can be divided into the following four categories: 1. C1, C2, C4 of classical activation pathways; 2. B factor, D factor, and P factor of the pathway activation pathway; 3. MBL and serine proteases in the activation pathway of mannan binding lectin (MBL); 4. C3, C5, C6, C7, C8, and C9 are involved in the common terminal pathway.

The complement activation process can be divided into three pathways based on its starting order: 1. The classical pathway starts from C1q-C1r2-C1s2, with antigen antibody complexes as the main activators; 2. The alternative pathway starting from C3 does not depend on antibodies; 3. The lectin activation pathway (MBL pathway) is recognized by mannan binding lectin (MBL) glycosylation. The above three pathways share a common terminal pathway, namely the formation of membrane attack complexes and their cell lysis effects.

The classical pathway of complement refers to the sequential activation of C1r, C1s, C4, C2 and C3 after the combination of C1q and immune complex, forming a cascade of enzymatic reactions between C3 convertase (C4b 2b) and C5 convertase (C4b2b3b). It is the main effect mode of humoral immune response mediated by antibody.

The immune complex is mainly composed of IgG and IgM molecules that bind to antigens. Each C1q molecule must bind to the Fc segment of two or more immunoglobulin molecules; Free or soluble antibodies cannot activate complement. The complement components involved in classical pathway activation are C1, C4, C2 and C3, C5-C9 in sequence.

The activation process of classical pathways can be divided into three main stages: 1. Identification stage; 2. Activation stage; 3. Membrane attack stage (membrane attack stage). During the activation process of the complement system, various bioactive substances can be produced, causing a series of biological effects. The biological effects of complement are: 1. Enhance phagocytosis and enhance the chemotaxis of phagocytic cells, 2. Increase the permeability of blood vessels, 3. Neutralize viruses, 4. Cytolytic effect, 5. Regulatory effect of immune response, etc.

Studies have shown that (Reference: Complement Is Activated by IgG Hexamers Assembled at the Cell Surface[J]. Science, 2014, 343(6176): 1260-3.), hexamer is the most potent form of binding C1q and initiating the classical activation pathway. Genmab developed the HexaBody platform based on this principle (references: Jong R, Beurskens F J, Verploegen S, et al. A Novel Platform for the Potentiation of Therapeutic Antibodies Based on Antigen-Dependent Formation of IgG Hexamers at the Cell Surface[J]. PLOS Biology, 2016, 14(1): e1002344.). The technical characteristics of this platform are: by inducing Fc specific site mutations (e.g., E345R), the interaction between Fc and Fc can be enhanced, which can promote the formation of hexamers after the antibody binds to cell surface antigens. The formation of hexamers can effectively enhance immune effector functions, especially to improve the complement-dependent cytotoxicity (CDC) of antibodies. However, the methods in the prior art are not yet satisfactory.

ADCC, which is antibody-dependent cell-medicated cytotoxicity (ADCC), refers to the Fab region of the antibody binding to the antigen on the surface of tumor cells or other target cells, and the Fc region mediates the killer cells to directly kill the target cells by binding to FcyR on the surface of killer cells (NK cells, macrophages, etc.). NK cells are the main effector cells of the antibody to play the role of ADCC, and when the Fc region of the antibody binds to the FcyR on the surface of NK cells, it will cause the latter to activate, and the activated NK cells will release cytotoxic substances such as perforin and granzyme to cause apoptosis of the target cells, so as to achieve the purpose of killing the target cells. It can be seen that the ADCC effect is also one of the main mechanisms by which related antibodies exert biological activity. The antibody subtype with the most developed ADCC activity in the current biopharmaceutical field is the wild-type IgG1.

Therefore, there is still a need to develop platform antibodies with good CDC and/or ADCC activity in this field.

### Summary of the invention

The object of the present invention is to provide a platform for enhancing the CDC activity of an antibody or fragment or fusion protein thereof, preferably a platform for enhancing the CDC and/or ADCC activity of an antibody or fragment or fusion protein thereof.

The first aspect of the present invention is to provide an antibody or fragment or fusion protein thereof with antigen-binding activity and CDC activity, and the antibody or fragment or fusion protein thereof comprises a binding functional domain targeting a predetermined antigen, and a heavy chain Fc region element, wherein the heavy chain Fc region element comprises:
(1) an amino acid residue at position 309 in the Fc region selected from the group consisting of: W, Y, E, F, H, C, D, N, Q, R, S, T, and K; and/or
(2) a tail-piece element at the C-terminus.

In another preferred embodiment, the heavy chain Fc region element is connected to the tail-piece element via a linker.

In another preferred embodiment, the heavy chain Fc region element comprises two heavy chain Fc regions, each of the heavy chain Fc regions comprises (1) an amino acid residue at position 309 in the Fc region selected from the group consisting of: W, Y, E, F, H, C, D, N, Q, R, S, T, and K; and/or
(2) a tail-piece element at the C-terminus.

In another preferred embodiment, each heavy chain Fc region further comprises R at position 345.

In another preferred embodiment, the fusion protein furhter has ADCC activity.

In another preferred embodiment, the binding functional domain targeting the predetermined antigen binds to an antigen molecule selected from thegroup consisting of: CD38, CD3, CD47, CD19, CD20, HER2, EGFR, CD123, Glypican-3, CD25, Trop-2, EpCAM, and a combination thereof.

In another preferred embodiment, the binding functional domain targeting the predetermined antigen comprises the following group of domains: VH, VL, Fab, F(ab')₂, Fab', scFv, or VHH.

In another preferred embodiment, the C-terminus of the CH3 domain of the heavy chain Fc region element comprises a tail-piece element.

In another preferred embodiment, the heavy chain Fc region element is derived from IgG.

In another preferred embodiment, the IgG has an amino acid sequence selected from the groupconsisting of: SEQ ID NO:31, SEQ ID NO:32, and SEQ ID NO:34.

In another preferred embodiment, the IgG is derived from mammals, preferably from rats, cynomolgus monkeys or humans, and preferably from humans.

In another preferred embodiment, the heavy chain Fc region element comprises CH2 and/or CH3 domains derived from IgG1, IgG2, IgG3, or IgG4.

In another preferred embodiment, the heavy chain Fc region element is derived from human IgG1.

In another preferred embodiment, the heavy chain Fc region element comprises CH2 and CH3 domains derived from IgG1.

In another preferred embodiment, the antibody or fragment or fusion protein thereof is a monospecific, bispecific, trispecific or multispecific antibody or fragment or fusion protein thereof.

In another preferred embodiment, the antibody or fragment or fusion protein thereof is dimer, trimer, or multimer.

In another preferred embodiment, the multimer is a hexamer.

In another preferred embodiment, the heavy chain Fc region element comprises an amino acid residue at position 309 in the Fc region selected from the group consisting of: F, C, W, and Y.

In another preferred embodiment, the heavy chain Fc region element further comprises amino acid substitutions at other positions.

In another preferred embodiment, the heavy chain Fc region element further comprises: a glutamic acid residue at position 345 of the Fc region is substituted by an arginine residue (E345R).

In another preferred embodiment, the heavy chain Fc region element comprises amino acid residue substitutions at positions in the Fc region, which are selected from the group consisting of:
(1)L309W+E345R;
(2)L309Y+E345R;
(3)L309E+E345R;
(4)L309F+E345R;
(5)L309H+E345R;
(6)L309C+E345R;
(7)L309D+E345R;
(8)L309N+E345R;
(9)L309Q+E345R;
(10)L309R+E345R;
(11)L309S+E345R;
(12)L309T+E345R; and
(13)L309K+E345R.

In another preferred embodiment, the heavy chain Fc region element comprises amino acid residue substitutions of L309W+E345R at positions in the Fc region.

In another preferred embodiment, the amino acid number of the heavy chain Fc region element is in accordance with the EU numbering system.

In another preferred embodiment, the tail-piece element is derived from human IgM.

In another preferred embodiment, the tail-piece element comprises one or more mutations of amino acids.

In another preferred embodiment, the tail-piece element sequence is as shown in SEQ ID NO: 7 or 8.

In another preferred embodiment, the binding functional domain targeting the predetermined antigen is a binding functional domain binding to CD38, which comprises a heavy chain variable region and a light chain variable region selected from the group consisting of:
(1) a heavy chain variable region, which comprises three complementary determinant regions (CDRs) as follows:
   H-CDR1 as shown in SEQ ID NO: 11,
   H-CDR2 as shown in SEQ ID NO: 12,
   H-CDR3 as shown in SEQ ID NO: 13, and
(2) a light chain variable region, which comprises three complementary determinant regions (CDRs) as follows:
   L-CDR1 as shown in SEQ ID NO: 14,
   L-CDR2 as shown in SEQ ID NO: 15,
   L-CDR3 as shown in SEQ ID NO: 16,
(2) a heavy chain variable region, which comprises three complementary determinant regions (CDRs) as follows:
   H-CDR1 as shown in SEQ ID NO: 35,
   H-CDR2 as shown in SEQ ID NO: 36,
   H-CDR3 as shown in SEQ ID NO: 37, and
(2) a light chain variable region, which comprises three complementary determinant regions (CDRs) as follows:
   L-CDR1 as shown in SEQ ID NO: 38,
   L-CDR2 as shown in SEQ ID NO: 39,
   L-CDR3 as shown in SEQ ID NO: 40.

In another preferred embodiment, the antibody or fragment or fusion protein thereof is selected from the group consisting of:
(a) an antibody or fragment or fusion protein thereof having amino acid sequences selected from the group consisting of:
   a heavy chain comprising a VH region containing H-CDR1 as shown in SEQ ID NO: 35, H-CDR2 as shown in SEQ ID NO: 36, and H-CDR3 as shown in SEQ ID NO: 37, and a heavy chain constant region with a W or Y mutation at position 309 of human IgG1 according to the EU numbering; and a light chain comprising a VL region containing L-CDR1 as shown in SEQ ID NO: 38, L-CDR2 as shown in SEQ ID NO: 39, and L-CDR3 as shown in SEQ ID NO: 40; or
   a heavy chain comprising a VH region containing H-CDR1 as shown in SEQ ID NO: 11, H-CDR2 as shown in SEQ ID NO: 12, and H-CDR3 as shown in SEQ ID NO: 13, and a heavy chain constant region with a W or Y mutation at position 309 of human IgG1 according to the EU numbering; and a light chain comprising a VL region containing L-CDR1 as shown in SEQ ID NO: 14, L-CDR2 as shown in SEQ ID NO: 15, and L-CDR3 as shown in SEQ ID NO: 16; or
   a heavy chain comprising a VH region containing H-CDR1 as shown in SEQ ID NO: 35, H-CDR2 as shown in SEQ ID NO: 36, and H-CDR3 as shown in SEQ ID NO: 37, and a heavy chain constant region with a W or Y mutation at position 309 and a R mutation at position 345 of human IgG1 according to the EU numbering; and a light chain comprising a VL region containing L-CDR1 as shown in SEQ ID NO: 38, L-CDR2 as shown in SEQ ID NO: 39, and L-CDR3 as shown in SEQ ID NO: 40; or
   a heavy chain comprising a VH region containing H-CDR1 as shown in SEQ ID NO: 11, H-CDR2 as shown in SEQ ID NO: 12, and H-CDR3 as shown in SEQ ID NO: 13, and a heavy chain constant region with a W or Y mutation at position 309 and a R mutation at position 345 of human IgG1 according to the EU numbering; and a light chain comprising a VL region containing L-CDR1 as shown in SEQ ID NO: 14, L-CDR2 as shown in SEQ ID NO: 15, and L-CDR3 as shown in SEQ ID NO: 16; or
   a heavy chain comprising a VH region containing H-CDR1 as shown in SEQ ID NO: 35, H-CDR2 as shown in SEQ ID NO: 36, and H-CDR3 as shown in SEQ ID NO: 37, and a heavy chain constant region as shown in SEQ ID NO: 31; and a light chain comprising a VL region containing L-CDR1 as shown in SEQ ID NO: 38, L-CDR2 as shown in SEQ ID NO: 39, and L-CDR3 as shown in SEQ ID NO: 40; or
   a heavy chain comprising a VH region containing H-CDR1 as shown in SEQ ID NO: 11, H-CDR2 as shown in SEQ ID NO: 12, and H-CDR3 as shown in SEQ ID NO: 13, and a heavy chain constant region as shown in SEQ ID NO: 31; and a light chain comprising a VL region containing L-CDR1 as shown in SEQ ID NO: 14, L-CDR2 as shown in SEQ ID NO: 15, and L-CDR3 as shown in SEQ ID NO: 16;
(b) a polypeptide derived from (a), that is formed by substitution, deletion, or addition of one or more amino acid residues to the amino acid sequence in (a), and has antigen binding function and CDC activity.

In another preferred embodiment, the antibody or fragment or fusion protein thereof is selected from the group consisting of:
(a) an antibody or fragment or fusion protein thereof comprising amino acid sequences selected from the group consisting of:
   a heavy chain comprising a VH region with the sequence as shown in SEQ ID NO:1, and a heavy chain constant region with a W or Y mutation at position 309 of human IgG1 according to the EU numbering; and a light chain comprising a VL region with the sequence as shown in SEQ ID NO: 2; or
   a heavy chain comprising a VH region with the sequence as shown in SEQ ID NO:21, and a heavy chain constant region with a W or Y mutation at position 309 of human IgG1 according to the EU numbering; and a light chain comprising a VL region with the sequence as shown in SEQ ID NO: 22; or
   a heavy chain comprising a VH region with the sequence as shown in SEQ ID NO: 1, and a heavy chain constant region with a W or Y mutation at position 309 and a R mutation at position 345 of human IgG1 according to the EU numbering; and a light chain comprising a VL region with the sequence as shown in SEQ ID NO: 2; or
   a heavy chain comprising a VH region with the sequence as shown in SEQ ID NO: 21, and a heavy chain constant region with a W or Y mutation at position 309 and a R mutation at position 345 of human IgG1 according to the EU numbering; and a light chain comprising a VL region with the sequence as shown in SEQ ID NO: 22; or
   a heavy chain comprising a VH region with the sequence as shown in SEQ ID NO: 1, and a heavy chain constant region with the sequence as shown in SEQ ID NO: 31; and a light chain comprising a VL region with the sequence as shown in SEQ ID NO: 2; or
   a heavy chain comprising a VH region with the sequence as shown in SEQ ID NO: 21, and a heavy chain constant region with the sequence as shown in SEQ ID NO: 31; and a light chain comprising a VL region with the sequence as shown in SEQ ID NO: 22;
(b) a polypeptide derived from (a), that is formed by substitution, deletion, or addition of one or more amino acid residues to the amino acid sequence in (a), and has antigen binding function and CDC activity.

In another preferred embodiment, the antibody or fragment or fusion protein thereof is selected from the group consisting of:
(a) an antibody or fragment or fusion protein thereof comprising amino acid sequences selected from the group consisting of:
   a heavy chain comprising a VH region with the sequence as shown in SEQ ID NO: 1, and a heavy chain constant region with the sequence as shown in SEQ ID NO: 31; and a light chain comprising a VL region with the sequence as shown in SEQ ID NO: 2; or
   a heavy chain comprising a VH region with the sequence as shown in SEQ ID NO: 21, and a heavy chain constant region with the sequence as shown in SEQ ID NO: 31; and a light chain comprising a VL region with the sequence as shown in SEQ ID NO: 22; or
   a heavy chain comprising a VH region with the sequence as shown in SEQ ID NO: 1, and a heavy chain constant region with the sequence as shown in SEQ ID NO: 32; and a light chain comprising a VL region with the sequence as shown in SEQ ID NO: 2; or
   a heavy chain comprising a VH region with the sequence as shown in SEQ ID NO: 21, and a heavy chain constant region with the sequence as shown in SEQ ID NO: 32; and a light chain comprising a VL region with the sequence as shown in SEQ ID NO: 22; or
   a heavy chain comprising a VH region with the sequence as shown in SEQ ID NO: 1, and a heavy chain constant region with the sequence as shown in SEQ ID NO: 34; and a light chain comprising a VL region with the sequence as shown in SEQ ID NO: 2; or
   a heavy chain comprising a VH region with the sequence as shown in SEQ ID NO: 21, and a heavy chain constant region with the sequence as shown in SEQ ID NO: 34; and a light chain comprising a VL region with the sequence as shown in SEQ ID NO: 22;
(b) a polypeptide derived from (a), that is formed by substitution, deletion, or addition of one or more amino acid residues to the amino acid sequence in (a), and has antigen binding function and CDC activity.

The second aspect of the present invention is to provide a method for improving the CDC of an antibody or fragment or fusion protein thereof, wherein the antibody or fragment or fusion protein thereof comprises a Fc region of immunoglobulin and a binding functional domain targeting a predetermined antigen, and the method comprises:
(S1a) Mutations in one or more amino acid residues are introduced into the antibody or fragment or fusion protein thereof, the mutation comprising that the amino acid at position 309 in the Fc region is mutated to an amino acid selected from the group consisting of: W, Y, E, F, H, C, D, N, Q, R, S, T, and K; and/or
(S1b) A tail-piece element is fused at the C-terminus of the Fc region, and the sequence of the tail-piece element is shown in SEQ ID NO: 7 or 8.

In another preferred embodiment, the L at position 309 in the Fc region of the IgG heavy chain is mutated into an amino acid selected from the group consisting of: W, Y, E, F, H, C, D, N, Q, R, S, T, and K.

In another preferred embodiment, the method further comprises:
(S2) Compare the CDC performance of a mutated antibody or fragment or fusion protein thereof with that of a pre-mutation antibody or fragment or fusion protein thereof.

In another preferred embodiment, the method further comprises a step of improving the ADCC activity of an antibody or fragment or fusion protein thereof.

In another preferred embodiment, (S1a) and (S1b) may be performed simultaneously, sequentially, or in reverse order.

In another preferred embodiment, the mutation comprises that the position 309 in the Fc region of the IgG heavy chain is mutated to an amino acid selected from the fgroup consisting of: W, Y.

In another preferred embodiment, in step (S1a), when the position 309 in the heavy chain Fc region of the antibody or fragment or fusion protein thereof is not Y and not W, it is mutated to Y or W.

In another preferred embodiment, in step (S1a), when the position 309 in the heavy chain Fc region of the antibody or fragment or fusion protein thereof is not Y, it is mutated to Y.

In another preferred embodiment, in step (S1a), when the position 309 in the heavy chain Fc region of the antibody or fragment or fusion protein thereof is not W, it is mutated to W.

In another preferred embodiment, the mutation further comprises that the position 345 in the Fc region of the IgG heavy chain is mutaed to R.

In another preferred embodiment, in step (S1a), the mutation comprises that the L at position 309 in the Fc region of the IgG heavy chain is mutated to an amino acid selected from the group consisting of: W, Y, E, F, H, C, D, N, Q, R, S, T, and K; and
the E at position 345 in the Fc region of the IgG heavy chain is mutated to R.

In the third aspect of the present invention, it provides a heavy chain Fc region element, which comprises:
(1) an amino acid residue at position 309 in the Fc region selected from the group consisting of: W, Y, E, F, H, C, D, N, Q, R, S, T, and K; and/or
(2) a tail-piece element at the C-terminus.

In another preferred embodiment, the heavy chain Fc region element is connected to the tail-piece element via a linker.

In another preferred embodiment, the C-terminus of the CH3 domain of the heavy chain Fc region element comprises a tail-piece element.

In another preferred embodiment, the heavy chain Fc region element is derived from IgG.

In another preferred embodiment, the heavy chain Fc region element is derived from human IgG1.

In another preferred embodiment, the heavy chain Fc region element comprises CH2 and/or CH3 domains derived from IgG1, IgG2, IgG3, or IgG4.

In another preferred embodiment, the heavy chain Fc region element comprises CH2 and CH3 domains derived from IgG1.

In another preferred embodiment, the heavy chain Fc region element comprises an amino acid residue at position 309 in the Fc region selected from the group consisting of: F, C, W, and Y.

In another preferred embodiment, the heavy chain Fc region element futher comprises amino acid substitutions at other positions.

In another preferred embodiment, the heavy chain Fc region element further comprises: a glutamic acid residue at position 345 of the Fc region is substituted by an arginine residue (E345R).

In another preferred embodiment, the heavy chain Fc region element comprises amino acid residue substitutions at positions in the Fc region, which are selected from the group consisting of:
(1)L309W+E345R;
(2)L309Y+E345R;
(3)L309E+E345R;
(4)L309F+E345R;
(5)L309H+E345R;
(6)L309C+E345R;
(7)L309D+E345R;
(8)L309N+E345R;
(9)L309Q+E345R;
(10)L309R+E345R;
(11)L309S+E345R;
(12)L309T+E345R; and
(13)L309K+E345R.

In another preferred embodiment, the amino acid number of the heavy chain Fc region element is in accordance with the EU numbering system.

In another preferred embodiment, the tail-piece element is derived from human IgM.

In another preferred embodiment, the tail-piece element comprises one or more mutations of amino acids.

In another preferred embodiment, the tail-piece element sequence is as shown in SEQ ID NO: 7 or 8.

In the fourth aspect of the present invention, it provides an isolated nucleic acid molecule encoding the antibody or fragment or fusion protein thereof of the first aspect of the present invention, or the heavy chain Fc region element of the third aspect of the present invention.

In the fifth aspect of the present invention, it provides an expression vector comprising the nucleic acid molecule of the fourth aspect of the present invention.

In the sixth aspect of the present invention, it provides a host cell comprising the expression vector of the the fifth aspect of the present invention.

In the seventh aspect of the present invention, it provides a method for preparing the antibody or fragment tor fusion protein thereof of the first aspect of the present invention, or the heavy chain Fc region element of the third aspect of the present invention, which comprises the following steps of:
(a) Under expression conditions, culturing the host cell of the sixth aspect of the present invention, thereby expressing the antibody or fragment or fusion protein thereof or the heavy chain Fc region element;
(b) Separating and purifying the antibody or fragment or fusion protein thereof or the heavy chain Fc region element as described in (a).

In the eighth aspect of the invention, it provides an immunoconjugate, which comprises:
(2) the antibody or fragment or fusion protein thereof of the first aspect of the present invention; and
(b) a coupling moiety selected from the group consisting of a detectable label, a drug, a toxin, a cytokine, a radionuclide, or an enzyme.

In the ninth aspect of the present invention, it provides a pharmaceutical composition comprising the antibody or fragment or fusion protein thereof of the first aspect of the present invention or the immunoconjugate of the eighth aspect of the present invention and a pharmaceutically acceptable carrier.

In the tenth aspect of the present invention, it provides a use of the antibody or fragment or fusion protein thereof of the first aspect of the present invention, or the immunoconjugate of the eighth aspect of the present invention, or the pharmaceutical composition of the ninth aspect of the present invention in the preparation of a drug for the treatment of cancer or immune-related diseases.

In another preferred embodiment, the cancer is selected from the group consisting of: melanoma, renal cancer, prostate cancer, pancreatic cancer, breast cancer, colon cancer, lung cancer, esophageal cancer, head and neck squamous cell cancer, liver cancer, ovarian cancer, cervical cancer, thyroid cancer, glioblastoma, glioma, multiple myeloma and other vegetative malignant diseases.

In another preferred embodiment, the immune-related disease is an autoimmune disease, preferably autoimmune nephropathy (immune nephritis, autoimmune kidney disease), lupus, systemic lupus erythematosus (SLE), Sjogren's syndrome, arthritis, rheumatoid arthritis, asthma, COPD, pelvic inflammatory disease, Alzheimer's disease, inflammatory bowel disease, Crohn's disease, ulcerative colitis, Peyronie's disease, chylous diarrhea, gallbladder disease, hair hiding disease, peritonitis, psoriasis, psoriasis arthritis, vasculitis, surgical adhesion, stroke, type I diabetes, Lyme disease, meningoencephalitis, autoimmune uveitis, multiple sclerosis, Guillain Barr syndrome, atopic dermatitis, autoimmune hepatitis, ankylosing spondylitis, fibrotic alveolitis, Grave's disease, idiopathic thrombocytopenic purpura (ITP), Meniere's disease, pemphigus, primary biliary cirrhosis, sarcoidosis, scleroderma, Wegener's granulomatosis, other autoimmune disorders, pancreatitis, trauma (surgery), graft-versus-host disease, transplant rejection, heart diseases (including ischemic diseases such as myocardial infarction and atherosclerosis), intravascular coagulation, bone resorption, osteoporosis, osteoarthritis, periodontitis and hypochloremia, infertility related to lack of fetal maternal tolerance, vitiligo, myasthenia gravis (MG), systemic sclerosis, inflammatory bowel disease, gastritis or IgG4 related diseases.

In another preferred embodiment, the immune-related disease is autoimmune nephropathy, preferably immunoglobulin A nephropathy (IgAN), membranous nephropathy (MN), or monoclonal gammopathy of renal significance (MGRS), lupus nephritis, or purpura nephritis.

In the eleventh aspect of the present invention, it provides a method for treating a disease, comprising a step of: administering the antibody or fragment or fusion protein thereof of the first aspect of the present invention, the pharmaceutical composition of the ninth aspect of the present invention, or the immunoconjugate of the eighth aspect of the present invention to a subject in need thereof.

In another preferred embodiment, the disease comprises cancer or immune-related diseases.

It should be understood that within the scope of the present invention, the various technical features of the present invention above and the various technical features specifically described hereinafter (as in the embodiments) may be combined with each other to constitute a new or preferred technical solution. Due to space limitations, it is not repeated here.

### Description of the figures

Figure 1 shows the CDC activity of antibodies containing site mutations at position 309 in the Fc segment (L309 E/F/H/C/D/N/Q/R/S/T/K/W/Y) on Raji cells.
Figure 2 shows the CDC activity of antibodies containing site mutations at position 309 in the Fc segment (L309 E/F/H/C/D/N/Q/R/S/T/K/W/Y) on Daudi cells.
Figure 3 shows the CDC activity of antibodies containing a site-directed mutation at Fc segment 309 (L309 F/C/W/Y) and antibodies modified at the end of the Fc segment (Pep and Pep-CS) against Audi cells.
Figure 4 shows the CDC activity of OKT10 Hu IgG1 and its mutants on Daudi cells.
Figure 5 shows the binding ability of 50G12 Hu IgG1, OKT10 Hu IgG1, Daratumumab IgG1, and Isatuximab IgG1 antibodies to cynomolgus monkeys CD38.
Figure 6 shows the killing activity of complement activation mediated by 50G12-L309W-E345R, single point mutant antibody 50G12-L309W, single point mutant antibody 50G12-E345R, unmutated antibody 50G12-Hu IgG1, and control antibody Daratumumab on Daudi cells.
Figure 7 shows the killing activity of complement activation mediated by 50G12-L309W-E345R, single point mutant antibody 50G12-L309W, single point mutant antibody 50G12-E345R, unmutated antibody 50G12-Hu IgG1, and control antibody Daratumumab on Romas cells.
Figure 8 shows the killing activity of complement activation mediated by 50G12-L309W-E345R, single point mutant antibody 50G12-L309W, single point mutant antibody 50G12-E345R, unmutated antibody 50G12-Hu IgG1, and control antibody Daratumumab on Raji cells.
Figure 9 shows the killing activity of ADCC effect mediated by 50G12-L309W-E345R, single point mutant antibody 50G12-L309W, single point mutant antibody 50G12-E345R, unmutated antibody 50G12-Hu IgG1, and control antibody Daratumumab on Daudi cells.
Figure 10 shows the killing activity of the ADCC effect mediated by 50G12-L309W-E345R, single point mutant antibody 50G12-L309W, single point mutant antibody 50G12-E345R, unmutated antibody 50G 12-Hu-IgG 1, and control antibody Daratumumab on NCI-H929 cells.
Figure 11 shows the killing activity of ADCC effect mediated by 50G12-L309W-E345R, unmutated antibody 50G 12-Hu-IgG 1, single point mutated antibody 50G12-L309W, single point mutated antibody 50G12-E345R and control antibody Daratumumab on Romas cells.
Figure 12 shows the killing activity of ADCC effect mediated by 50G12-L309W-E345R, single point mutant antibody 50G12-L309W, single point mutant antibody 50G12-E345R, unmutated antibody 50G 12-Hu-IgG 1 and control antibody Daratumumab on MOLP8 cells.
Figure 13 shows the killing activity of ADCC effect mediated by 50G12-L309W-E345R, single point mutant antibody 50G12-L309W, single point mutant antibody 50G12-E345R, unmutated antibody 50G12-Hu IgG1, and control antibody Daratumumab on Daudi cells.
Figure 14 shows the apoptosis-inducing effects of 50G12-L309W-E345R, unmutated antibody 50G12-Hu-IgG1, and control antibody Daratumumab.

### Detailed description

After extensive and intensive research, the inventor accidentally discovered for the first time that modifying the L309 position of the Fc of the constant region of the antibody heavy chain (preferably L309 and E345 positions) and/or fusing the tail-piece of human IgM in the Fc segment of the constant region of the antibody heavy chain can significantly enhance the immune effector function of the antibody, especially to improve the complement-dependent cytotoxicity (CDC) and antibody-dependent cell-mediated cytotoxicity (ADCC) of the antibody. On this basis, the present invention has been completed.

### The terms

As used herein, the term "antibody (Ab)" or "immunoglobulin (IgG)" is a heterotetrameric glycoprotein with the same structural characteristics, which consists of two identical light chains (L) and two identical heavy chains (H). Each light chain is connected to the heavy chain through a covalent disulfide bond, and the numbers of disulfide bonds between heavy chains of different immunoglobulin isotypes are different. Each heavy and light chain also has regularly spaced intrachain disulfide bonds. Each heavy chain has a variable region (VH) at one end, followed by constant regions, which consists of three domains CH1, CH2, and CH3. Each light chain has a variable region (VL) at one end and a constant region at the other end; the constant region of the light chain is paired to the first constant region of the heavy chain, and the variable region of the light chain is paired to the variable region of the heavy chain. The constant regions are not directly involved in the binding of antibodies to antigens, but they exhibit different effector functions, such as participating in antibody-dependent cell-mediated cytotoxicity (ADCC). The heavy chain constant region includes IgG1, IgG2, IgG3, IgG4 subtypes; The light chain constant region includes κ (Kappa) or λ (Lambda). The heavy and light chains of the antibody are covalently linked together by disulfide bonds between the CH1 domain of the heavy chain and the CL domain of the light chain, and the two heavy chains of the antibody are covalently linked together by the polypeptide disulfide bonds formed between the hinge regions. In the present invention, the terms "Fab" and "Fc" refer to papain may cleave the antibody into two identical Fab segments and one Fc segment. The Fab segment consists of VH and CH1 of the heavy chain of the antibody and VL and CL domains of the light chain. The Fab segment is fragment crystallizable (Fc), which consists of CH2 and CH3 domains of the antibody. The Fc segment has no antigen-binding activity and is the site of interaction between antibodies and effector molecules or cells. In the present invention, the term "variable" means that certain parts of the variable region of an antibody differ in sequence, which forms the binding and specificity of various specific antibodies for their specific antigens. However, the variability is not evenly distributed throughout the variable region of the antibody. It is concentrated in three segments called complementary determining regions (CDRs) or hypervariable regions in the light chain and heavy chain variable regions. The more conserved part of the variable region is called the framework region (FR). The variable regions of the natural heavy and light chains each contain four FR regions, which are roughly in the β-folded configuration, connected by the three CDRs that form the connecting loop, and in some cases may form a partly β folded structure. The CDRs in each chain get close through the FR regions and together with the CDRs of the other chain form the antigen-binding site of the antibody (see Kabat et al., NIH Publ. No. 91-3242, Volume I, pages 647-669 (1991)).

As used herein, the term "framework region (FR)" refers to amino acid sequences inserted between CDRs, i.e., those portions of the relatively conserved light and heavy chain variable regions of immunoglobulins between different immunoglobulins in a single species. The light and heavy chains of immunoglobulins each have four FRs, which are called FR1-L, FR2-L, FR3-L, FR4-L and FR1-H, FR2-H, FR3-H, FR4-H. Accordingly, the light chain variable domain may thus be referred to as (FR1-L)-(CDR1-L)-(FR2-L)-(CDR2-L)-(FR3-L)-(CDR3-L)-(FR4-L) and the heavy chain variable domain may thus be represented as (FR1-H)-(CDR1-H)-(FR2-H)-(CDR2-H)-(FR3-H)-(CDR3-H)-(FR4-H). Preferably, the FR of the present invention is a human antibody FR or its derivatives, the derivative of the human antibody FR is substantially the same as the naturally occurring human antibody FR, i.e., sequence identity reaches 85%, 90%, 95%, 96%, 97%, 98% or 99%.

Knowing the amino acid sequence of the CDR, those skilled in the art can easily determine the frame region FR1-L, FR2-L, FR3-L, FR4-L and/or FR1-H, FR2-H, FR3-H, FR4-H.

As used herein, the term "human frame region" is substantially the same (about 85% or more, specifically 90%, 95%, 97%, 99% or 100%) framework region as the naturally occurring human antibody framework region.

As used herein, "tail-piece element" and "TP element" can be used interchangeably, both referring to tail-piece sequences from IgM, preferably from human IgM. The TP element of the present invention may comprise any suitable amino acid sequence. The TP element may be a tail-piece found in a naturally occurring antibody or, optionally, a modified tail-piece sequence that differs in length and/or composition from the natural tail-piece. The modification can be a mutation of one or more amino acids, such as a mutation of cysteine in the tail-piece of the IgM to serine.

In another preferred example, the tail-piece element sequence is as shown in SEQ ID NO: 7 or 8.

It should be understood that the TP element can be directly fused with the C-terminal of the heavy chain Fc region. Alternatively, a short linker sequence can be provided between the TP element and the heavy chain Fc region.

As used herein, the term "linker" refers to a short linker sequence between the Fc region and the TP element, preferably a flexible linker. Examples of suitable linkers include monoglycine (Gly), or serine (Ser) residues, and the identification and sequence of amino acid residues in the linker may vary with the type of secondary structural element to be implemented in the linker.

### Antibody or their fragment or fusion protein

The antibody or its fragment or fusion protein of the invention is an antibody or its fragment or fusion protein with antigen-binding activity and CDC activity, comprising a binding functional domain targeting a predefined antigen, and a heavy chain Fc region element comprising two heavy chain Fc regions: Each heavy chain Fc region (1) contains an amino acid residue selected from the following group at the 309 position of Fc region: W, Y, E, F, H, C, D, N, Q, R, S, T, or K; And/or (2) contains a tail-piece element at the C-terminal. As used herein, the "heavy chain Fc region element" contains two heavy chain Fc regions. Unless otherwise specified, the mutation sites in the heavy chain Fc region elements of the present invention refer to the mutation sites contained on each heavy chain Fc region element in the heavy chain Fc region elements.

It should be understood that the specific CDC enhanced mutation at position 309 of the heavy chain Fc region element of the antibody or its fragment or fusion protein of the present invention can be combined with other techniques that enhance antibody performance (such as CDC, specificity, affinity, etc.), such as binding to E345R mutations. Preferably, when each heavy chain Fc region element of the antibody or its fragment or fusion protein in the present invention contains a mutation combination L309W+E345R, it has an unexpected significant enhancement effect on CDC; more preferably, it has a better effect on significantly enhancing the activity of CDC and ADCC.

As used herein, the terms "CDC platform antibody", "antibody of the invention", "antibody of the invention or its fragment", "fusion protein of the invention", "antibody of the invention or its fragment or fusion protein" are used interchangeably to refer to the CDC-enhanced antibody or its fragment or fusion protein described in the first part of the invention. It should be understood that the terms also include active fragments of antibodies or fragments thereof or fusion proteins of the present invention, which retain both antigen binding activity and specific mutations and/or TP (tail-piece) element at position 309.

In this manual, unless otherwise specified, the EU numbering system is used to refer to residues in the antibody domain. The system was originally designed by Edelman et al. in 1969 and described in detail in the following literature: Kabat et al., 1987 (Edelman et al., 1969; "The covalent structure of an entire γG immunoglobulin molecule," PNAS Biochemistry vol. 63 pp78-85. Kabat et al.,1987; Sequences of Proteins of Immunological Interest, US Department of Health and Human Services, NIH, USA). When assigning a position number and/or amino acid residue to a specific antibody isotype, it is known to those skilled in the art that it is intended to be applicable to the corresponding position and/or amino acid residue of any other antibody isotype. For example, the wild-type residues found at position 309 in naturally occurring human IgG1, IgG3, and IgG4 are leucine residues, while the wild-type residues found in naturally occurring IgG2 are valine residues. When referring to amino acid residues originating from the tail of IgM or IgA, according to conventional practice in this field, the given position number is the position number of naturally occurring residues in IgM or IgA. As used in this article, "amino acid residue at position 309" refers to the residue at position 309 in the naturally occurring human IgG1, and "amino acid residue at position 345" refers to the residue at position 345 in the naturally occurring human IgG1.

It should be understood that the present invention also provides a method for constructing CDC platform antibodies or fragments thereof or fusion proteins, that is, a method for constructing antibodies or fragments thereof or fusion proteins of the present invention, as described in the second aspect of the present invention. The method of constructing CDC platform antibodies, fragments thereof, or fusion proteins according to the present invention can enhance the CDC activity of antibodies targeting predetermined antigens, fragments thereof, or fusion proteins, and further significantly enhance the CDC and/or ADCC activity of fusion proteins.

In the present invention, the antibody or its fragment or fusion protein contains a binding functional domain targeting a predetermined antigen that can target different antigenic targets, and the binding functional domain targeting the predetermined antigen can bind to an antigen molecule selected from the following group: CD38, CD3, CD47, CD19, CD20, HER2, EGFR, CD123, Glypican-3, CD25, Trop-2, EpCAM, or a combination thereof.

Taking CD38 as an example, preferably, the sequence of the anti-CD38 antibody of the invention is described in patent application CN202010805420.2, and persons skilled in the art may also modify or modify the binding functional domain of the antigen of the invention by means of techniques well known in the art, such as adding, deletion and/or substituting one or more amino acid residues. Thus, the affinity or structural stability of the binding functional domain of the antigen can be further increased, and the results after modification or modification can be obtained by conventional determination methods.

In the present invention, the antibody or its fragment or fusion protein also comprises the conservative variant of the antibody of the invention thereof means that compared with the amino acid sequence of the antibody of the present invention, there are at most 10, preferably at most 8, more preferably at most 5, and preferably at most 3 amino acids are replaced by amino acids with the same or similar properties to form a polypeptide. These conservative variant polypeptides are best produced by amino acid substitution according to Table A.

**Table A**

| Initial residue | Representative substitution | Preferred substitution |
|---|---|---|
| Ala (A) | Val; Leu; Ile | Val |
| Arg (R) | Lys; Gln; Asn | Lys |
| Asn (N) | Gln; His; Lys; Arg | Gln |
| Asp (D) | Glu | Glu |
| Cys (C) | Ser | Ser |
| Gln (Q) | Asn | Asn |
| Glu (E) | Asp | Asp |
| Gly (G) | Pro; Ala | Ala |
| His (H) | Asn; Gln; Lys; Arg | Arg |
| Ile (I) | Leu; Val; Met; Ala; Phe | Leu |
| Leu (L) | Ile; Val; Met; Ala; Phe | Ile |
| Lys (K) | Arg; Gln; Asn | Arg |
| Met (M) | Leu; Phe; Ile | Leu |
| Phe (F) | Leu; Val; Ile; Ala; Tyr | Leu |
| Pro (P) | Ala | Ala |
| Ser (S) | Thr | Thr |
| Thr (T) | Ser | Ser |
| Trp (W) | Tyr; Phe | Tyr |
| Tyr (Y) | Trp; Phe; Thr; Ser | Phe |
| Val (V) | Ile; Leu; Met; Phe; Ala | Leu |

In the present invention, the terms "anti" and "binding" refers to a non-random binding reaction between two molecules, such as a reaction between an antibody and an antigen targeted thereto. Typically, antibody binds to this antigen with an equilibrium dissociation constant (KD) of less than about 10⁻⁷M, e.g. less than about 10⁻⁸M, 10⁻⁹M, 10⁻¹⁰M, 10⁻¹¹M, or less. The term "KD" refers to the equilibrium dissociation constant of a particular antibody-antigen interaction, which is used to describe the binding affinity between the antibody and the antigen. The smaller the equilibrium dissociation constant, the closer the antibody-antigen binding and the higher the affinity between the antibody and the antigen. For example, the binding affinity of an antibody to an antigen is determined using Surface Plasmon Resonance (SPR) in a BIACORE apparatus or the relative affinity of an antibody binding to an antigen is determined using an ELISA.

The antibody of the present invention or a fragment or fusion protein thereof may be used alone or in combination or conjugated with a detectable marker (for diagnostic purposes), a therapeutic agent, or any combination of any of the above

### Coding nucleic acid and expression vector

The present invention also provides the polynucleotide molecule encoding the antibody above mentioned or fragment thereof or fusion protein thereof. The polynucleotide of the present invention may be in the form of DNA or RNA. DNA form includes cDNA, genomic DNA, or synthetic DNA. DNA may be single-stranded or double-stranded. DNA may be a coding strand or a non-coding strand. In the present invention, the term "expression vector" means a vector carrying an expression box for expressing a specific purpose protein or other substance, such as a plasmid, viral vector (e.g., adenovirus, retrovirus), phage, yeast plasmid, or other vectors. For example, conventional expression vectors in the art containing appropriate regulatory sequences, such as promoters, terminators, enhancers, etc., including but not limited to: viral vectors (such as adenovirus, retrovirus), plasmids, bacteriophages, yeast plasmids, or other vectors. The expression vector preferably includes pDR1, pcDNA3.4 (+), pDHFR or pTT5.

Once a relevant sequence is obtained, recombination methods can be used to obtain the relevant sequence in large quantities. This is usually carried out by cloning the sequence into a vector, transforming a cell with the vector, and then separating the relevant sequence from the proliferated host cell by conventional methods.

The present invention further relates to a vector comprising said suitable DNA sequence and a suitable promoter or a control sequence. These vectors can be used to transform suitable host cells to enable them to express protein.

In the present invention, the term "host cell" refers to a variety of host cells that are conventional in the art, as long as the vector can be stably replicated on its own, and the polynucleotide molecules carried can be efficiently expressed. The host cell comprises a prokaryotic expression cell and a eukaryotic expression cell, and the host cell preferably comprises: COS, CHO, NS0, sf9, sf21, DH5α, BL21 (DE3), TG1, BL21 (DE3), 293F or 293E cells

### Pharmaceutical Composition

The present invention further provides a composition. Preferably, the composition is a pharmaceutical composition comprising the antibody above mentioned, or an active fragment thereof, or a fusion protein thereof, and a pharmaceutically acceptable carrier. Typically, these substances may be formulated in a non-toxic, inert and pharmaceutically acceptable aqueous carrier medium, wherein the pH is typically about 4-8 and preferably about 5-7, although the pH may vary depending on the nature of the substance being formulated and the condition to be treated. The formulated pharmaceutical composition may be administered by conventional routes, including (but not limited to): intravenous injection, intravenous drip, subcutaneous injection, local injection, intramuscular injection, intratumoral injection, intraperitoneal injection (e.g., intraperitoneal), intracranial injection, or intraluminal injection.

In the present invention, the term "pharmaceutical composition" refers to the fact that the bifunctional antibody or its fragment or fusion protein of the present invention can be combined with a pharmaceutically acceptable carrier to form a pharmaceutical preparation composition so as to exert a more stable curative effect, and these preparations can ensure the conformational integrity of the amino acid core sequence of the bifunctional antibody or its fragment or fusion protein disclosed in the present invention, and also protect the polyfunctional groups of the protein from its degradation (including but not limited to coagulation, deamination or oxidation).These preparations can ensure the conformational integrity of the amino acid core sequence of the bifunctional antibody or its fragment or fusion protein disclosed in the present invention, while also protecting the protein multifunctional groups to prevent their degradation (including but not limited to coagulation, deamination or oxidation).

The pharmaceutical composition according to the present invention comprises a safe and effective amount (e.g., 0.001-99 wt %, preferably 0.01-90 wt %, preferably 0.1-80 wt %) of the bifunctional antibody or its fragment or fusion protein (or a conjugate thereof) of the present invention and a pharmaceutically acceptable carrier or excipient. Such carriers include (but are not limited to): saline, buffers, glucose, water, glycerol, ethanol, and a combination thereof. Pharmaceutical preparations should correspond to the administration modes. The pharmaceutical composition according to the present invention can be prepared in the form of an injection, for example, by a conventional method using physiological saline or an aqueous solution containing glucose and other adjuvants. A pharmaceutical composition, for example, an injection and a solution, should be prepared under aseptic conditions. The dosage of the active ingredient is a therapeutically effective amount, for example, about 10 µg/kg body weight per day to about 50 mg/kg body weight. In addition, the bifunctional antibody or its fragment or fusion protein of the present invention may also be used in combination with an additional therapeutic agent.

When a pharmaceutical composition is used, a safe and effective amount of a bifunctional antibody or its fragment or fusion protein or immunoconjugate thereof is administered to a mammal, wherein the safe and effective amount is generally at least about 10 µg per kilogram of body weight, and in most cases, no more than about 50 mg per kilogram of body weight, preferably, the amount is from about 10 µg per kilogram of body weight to about 10 mg per kilogram of body weight. Of course, a specific amount should also depend on the factors such as administration route and physical conditions of a patient, which fall into the skills of skilled physicians.

In the present invention, the term "effective amount" means the amount or dose of the pharmaceutical composition of the present invention, after administration to a subject, that produces the desired effect in the treated individual, including the improvement of the individual's condition. The term "subject" includes, but is not limited to, mammals, such as humans, non-human primates, rats, and mice.

### Uses

The present invention also provides uses for antibodies or their fragments or fusion proteins as described in the first aspect of the invention, or immune couplings as described in the eighth aspect of the invention, or pharmaceutical compositions as described in the ninth aspect of the invention, such as for the preparation of diagnostic preparations or the preparation of pharmaceuticals.

Preferably, the drug is a drug used to prevent and/or treat cancer, or immune-related diseases.

In present invention, the cancer is selected from the following groups: melanoma, renal cancer, prostate cancer, pancreatic cancer, breast cancer, colon cancer, lung cancer, esophageal cancer, head and neck squamous cell cancer, liver cancer, ovarian cancer, cervical cancer, thyroid cancer, glioblastoma, glioma, multiple myeloma and other vegetative malignant diseases.

In a preferred embodiment of the present invention, the drugs are drugs used to prevent and/or treat a disease associated with abnormal expression or function of CD38.

In the present invention, the diseases associated with abnormal CD38 expression or function are conventional diseases associated with abnormal CD38 expression or function in the art. Preferably, the diseases associated with abnormal CD38 expression or function are tumors/cancers, or immune-related diseases.

Preferably, the disease is preferred as an immune related disease, such as an autoimmune disease. More preferably, the drug is used to treat and/or prevent autoantibody-mediated autoimmune diseases. Further, the autoimmune diseases include autoimmune nephropathy (immune nephritis, autoimmune nephropathy), lupus, systemic lupus erythematosus (SLE), Graves' disease, myasthenia gravis (MG), idiopathic thrombocytopenic purpura (ITP), autoimmune nephropathy (immune nephritis, autoimmune nephropathy), lupus, systemic lupus erythematosus (SLE), Sjogren's syndrome, Arthritis, Rheumatoid Arthritis, Asthma, COPD, Pelvic Inflammatory Disease, Alzheimer's Disease, Inflammatory Bowel Disease, Crohn's Disease, Ulcerative Colitis, Peyronie's Disease, Celiac Disease, Gallbladder Disease, Pilonidal Disease, Peritonitis, Psoriasis, Psoriatic Arthritis, Vasculitis, Surgical Adhesions, Stroke, Type I Diabetes, Lyme disease, meningoencephalitis, autoimmune uveitis, multiple sclerosis, Guillain-Barr syndrome, atopic dermatitis, autoimmune hepatitis, ankylosing spondylitis, fibrotic alveolitis, Grave's disease, idiopathic thrombocytopenic purpura (ITP), Meniere's disease, pemphigus, Primary biliary cirrhosis, sarcoidosis, scleroderma, Wegener's granulomatosis, other autoimmune disorders, pancreatitis, trauma (surgery), graft-versus-host disease, transplant rejection, cardiac disease (including ischemic diseases such as myocardial infarction and atherosclerosis), intravascular coagulation, bone resorption, osteoporosis, osteoarthritis, periodontitis and hypochloremia, infertility associated with lack of fetal-maternal tolerance, vitiligo, myasthenia gravis (MG), systemic sclerosis, inflammatory bowel disease, gastritis, or IgG4-related disease, preferably, immunoglobulin A nephropathy (IgAN), membranous nephropathy (MN), or monoclonal gammopathy of renal significance (MGRS), lupus nephritis, or purpura nephritis due to the formation of immune complexes of plasma cell hyperproliferation, excessive secretion of autoantibodies, and immunoglobulins.

At present, there is still a significant clinical demand for the treatment of IgAN, MN, and MGRS, which are three types of autoimmune kidney diseases. In particular, specific targeted therapies are severely lacking. Only rituximab targeting CD20 is approved for the treatment of MN. However, due to the fact that CD20 is only expressed on activated B cells that produce fewer autoantibodies, the survey results show that up to 40% of MN patients in clinical practice are still ineffective against CD20 treatment. CD38 is highly expressed on plasma cells, and antibodies specifically targeting CD38 in this invention (such as 50G12-L309W-E345R, etc.) can cause plasma cell lysis and apoptosis through ADCC, ADCP, and CDC effects, effectively used for the treatment of IgAN, MN, MGRS, and multiple myeloma (MM).

### The main advantages of the present invention include:

(1) The present invention provides a novel technique for enhancing CDC and/or ADCC. This technology has the potential to transform antibody drugs with limited or missing CDC and/or ADCC activity into antibody drugs with enhanced CDC and/or ADCC activity. By using this technology, many ready-made antibodies that act on different targets/target cells can be directly modified. By using this technology, the efficacy of existing antibodies can be improved and various diseases can be treated.
(2) The CDC and/or ADCC performance of the antibody or its fragment or fusion protein of the present invention can be significantly improved by site-directed mutation of amino acid at position 309. It should be understood that the specific CDC enhanced mutation at position 309 of the heavy chain Fc region element of the antibody or its fragment or fusion protein of the present invention can be combined with other techniques that enhance antibody performance (such as CDC, specificity, affinity, etc.), such as binding to E345R mutations.

The invention is further illustrated below in conjunction with specific embodiments. It should be understood that the examples are not intended to limit the scope of the invention. The conditions of the experimental methods not specifically indicated in the following examples are usually in accordance with conventional conditions as described in Sambrook et al., Molecular Cloning: A Laboratory Manual (New York: Cold Spring Harbor Laboratory Press, 1989), or according to the conditions recommended by the manufacturer. Unless otherwise stated, percentages and parts are calculated by weight.

### Related reagent consumables:

EZ-link NHS-LC-Biotin (Thermo Fisher Scientific, No. 21343); Biotin CAPture Kit, Series S (Cytiva, No. 28920234); HBS-EP+ pH7.4 buffer (GE Healthcare, No. BR-1006-69); Daudi, Raji, Romas, NCI-H929 cells were purchased from ATCC(American Type Culture Collection), MOLP8 cells were purchased from Nanjing Kebai Biological Co., LTD. ADCC Bioassay effector cells (Suzhou Ruian Biotechnology Co., LTD., No. RA-CK01); Cell Titer-Glo Luminescent Assay (Promega, No. G7570); Normal Human Serum complement (Quidel Corporation, No. A11); Bio-Glo Luciferase Assay System (Promega, No. G7940); Annexin V-FITC/PI Apoptosis Detection Kit (Yeasen, No. 40302ES60).

The protein expression and purification methods used in the embodiments are described below:
The target gene is constructed into the expression vector pcDNA3.4, and the constructed expression vector or a combination of expression vectors are transferred into FreeStyle^{™} 293-F Cells cells (hereinafter referred to as HEK293F, purchased from Thermo Fisher Scientific) by using PEI (Polyethylenimine). HEK293F cells were cultured in Free Style 293 Expression Medium (purchased from Thermo Fisher Scientific) for 5 days, cell supernatants were collected, and antibodies were purified by Protein A affinity chromatography.

The ELISA detection methods used in the following embodiments are described as follows:
The microplates were coated with corresponding recombinant proteins and closed with PBST containing 1% bovine serum albumin (PBST is a phosphate buffer containing 0.05% Tween-20). The tested antibody was diluted in a gradient and it was transferred to a microplate coated with recombinant protein. The sample was incubated at room temperature for half an hour. After washing the plate, appropriately diluted HRP (Horseradish Peroxidase) labeled sheep anti human antibody (Fc specific, purchased from Sigma) was added and incubated at room temperature for half an hour. After washing, 100µL color developing solution with TMB(3,3 ',5,5 '-Tetramethylbenzidine) as substrate was added to each well and incubated at room temperature for 1 ~ 5 min. The reaction was terminated by adding 50 µL termination solution (2 M H₂SO₄). The board reader (SpectraMax 190) reads OD450. GraphPad Prism7 was used for mapping and data analysis, and EC50/IC50 was calculated.

The description of the physical and chemical property detection method used in the following embodiments is as follows:

### HPLC-SEC

Antibodies are high molecular weight proteins with highly complex secondary and tertiary structures. Due to post-translational modifications, aggregation, and degradation, antibodies exhibit heterogeneity in terms of biochemical and biophysical properties. When trispecific antibodies are analyzed by isolation techniques, variants, aggregates, and degraded fragments are often observed, and their presence may compromise safety and efficacy. During the production and storage of antibodies, aggregates, degraded fragments, and incomplete assembly of molecules are prone to occur. The present invention uses high-performance liquid chromatography size exclusion chromatography (HPLC-SEC) to detect the content of the above-mentioned impurities in the sample. The molecular weight of aggregates is greater than that of monomers, so the retention time of corresponding peaks is shorter; The molecular weight of degraded fragments or partially assembled molecules is smaller than that of monomers, therefore the retention time of corresponding peaks is longer. The chromatograph used for HPLC-SEC is Dionex Ultimate 3000; The preparation method for mobile phase was as follows: Take an appropriate amount of 20mM sodium dihydrogen phosphate mother liquor, and the pH was adjusted to 6.8 ± 0.1 with 20 mM disodium hydrogen phosphate; Injection volume: 20 µ g; The chromatographic column was TSK G3000SWXL, with a specification of 7.8 × 300 mm 5 µ M; Flow rate was 0.5 mL/min, elution time was 30 minutes; Column temperature was 25 °C, sample room temperature was 10 °C; The detection wavelength was 214nm.

### Antibody Sequences of the present invention

**Table 1 Antibody Sequences**

| names | Amino acid sequence | SEQ ID NO. |
|---|---|---|
| 50G12-Hu-I gG1 heavy chain variable region | QVQLVQSGAEVKKPGASVKVSCKASGYTFNTYWMQWVRQAPGQGLE WMGAIYPGDGDITYNQKFKGRVTLTADKSTSTVYMELSSLRSEDTAVY YCAREGYYYGGALDYWGQGTLVTVSS | 1 |
| 50G12-Hu-I gG1 light chain variable region | DIQMTQSPSSLSASVGDRVTITCTASSSVSSSYLHWYQQKPGKAPKLWM YGTSNLASGVPSRFSGSGSGTDYTLTISSLQPEDFATYYCHRYHRSPWTF GQGTKVEIK | 2 |
| Human IgG1 heavy chain constant region | ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKKV EPKSCDKTHTCPPCPAPELLGGPSVFLFPPKPKDTLMISRTPEVTCVVVD VSHEDPEVKFNWYVDGVEVHNAKTKPREEQYNSTYRVVSVLTVLHQD WLNGKEYKCKVSNKALPAPIEKTISKAKGQPREPQVYTLPPSREEMTKN QVSLTCLVKGFYPSDIAVEWESNGQPENNYKTTPPVLDSDGSFFLYSKL TVDKSRWQQGNVFSCSVMHEALHNHYTQKSLSLSPGK | 3 |
| Human Kappa light chain constant region | | 4 |
| 50G12-Hu-I gG1 heavy chain | QVQLVQSGAEVKKPGASVKVSCKASGYTFNTYWMQWVRQAPGQGLE WMGAIYPGDGDITYNQKFKGRVTLTADKSTSTVYMELSSLRSEDTAVY YCAREGYYYGGALDYWGQGTLVTVSSASTKGPSVFPLAPSSKSTSGGT AALGCLVKDYFPEPVTVSWNSGALTSGVHTFPAVLQSSGLYSLSSVVTV PSSSLGTQTYICNVNHKPSNTKVDKKVEPKSCDKTHTCPPCPAPELLGGP SVFLFPPKPKDTLMISRTPEVTCVVVDVSHEDPEVKFNWYVDGVEVHN AKTKPREEQYNSTYRVVSVLTVLHQDWLNGKEYKCKVSNKALPAPIEK TISKAKGQPREPQVYTLPPSREEMTKNQVSLTCLVKGFYPSDIAVEWES NGQPENNYKTTPPVLDSDGSFFLYSKLTVDKSRWQQGNVFSCSVMHEA LHNHYTQKSLSLSPGK | 5 |
| 50G12-Hu-I gG1 light chain | DIQMTQSPSSLSASVGDRVTITCTASSSVSSSYLHWYQQKPGKAPKLWM [?]YGTSNLASGVPSRFSGSGSGTDYTLTISSLQPEDFATYYCHRYHRSPWTF GQGTKVEIKRTVAAPSVFIFPPSDEQLKSGTASVVCLLNNFYPREAKVQ WKVDNALQSGNSQESVTEQDSKDSTYSLSSTLTLSKADYEKHKVYACE VTHQGLSSPVTKSFNRGEC | 6 |
| Tail-piece of IgM | PTLYNVSLVMSDTAGTCY | 7 |
| Pep-CS | PTLYNVSLVMSDTAGTSY | 8 |
| 50G12-Hu-I gG1-Pep heavy chain | QVQLVQSGAEVKKPGASVKVSCKASGYTFNTYWMQWVRQAPGQGLE WMGAIYPGDGDITYNQKFKGRVTLTADKSTSTVYMELSSLRSEDTAVY YCAREGYYYGGALDYWGQGTLVTVSSASTKGPSVFPLAPSSKSTSGGT AALGCLVKDYFPEPVTVSWNSGALTSGVHTFPAVLQSSGLYSLSSVVTV PSSSLGTQTYICNVNHKPSNTKVDKKVEPKSCDKTHTCPPCPAPELLGGP SVFLFPPKPKDTLMISRTPEVTCVVVDVSHEDPEVKFNWYVDGVEVHN AKTKPREEQYNSTYRVVSVLTVLHQDWLNGKEYKCKVSNKALPAPIEK TISKAKGQPREPQVYTLPPSREEMTKNQVSLTCLVKGFYPSDIAVEWES NGQPENNYKTTPPVLDSDGSFFLYSKLTVDKSRWQQGNVFSCSVMHEA LHNHYTQKSLSLSPGKPTLYNVSLVMSDTAGTCY | 9 |
| 50G12-Hu-I gG1-Pep-CS heavy chain | QVQLVQSGAEVKKPGASVKVSCKASGYTFNTYWMQWVRQAPGQGLE WMGAIYPGDGDITYNQKFKGRVTLTADKSTSTVYMELSSLRSEDTAVY YCAREGYYYGGALDYWGQGTLVTVSSASTKGPSVFPLAPSSKSTSGGT AALGCLVKDYFPEPVTVSWNSGALTSGVHTFPAVLQSSGLYSLSSVVTV PSSSLGTQTYICNVNHKPSNTKVDKKVEPKSCDKTHTCPPCPAPELLGGP SVFLFPPKPKDTLMISRTPEVTCVVVDVSHEDPEVKFNWYVDGVEVHN AKTKPREEQYNSTYRVVSVLTVLHQDWLNGKEYKCKVSNKALPAPIEK TISKAKGQPREPQVYTLPPSREEMTKNQVSLTCLVKGFYPSDIAVEWES NGQPENNYKTTPPVLDSDGSFFLYSKLTVDKSRWQQGNVFSCSVMHEA LHNHYTQKSLSLSPGKPTLYNVSLVMSDTAGTSY | 10 |
| H-CDR1 of OKT10 heavy chain | RSWMN | 11 |
| H-CDR2 of OKT10 heavy chain | EINPDSSTINYTTSLKD | 12 |
| H-CDR3 of OKT10 heavy chain | YGNWFPY | 13 |
| L-CDR1 of the OKT10 light chain | KASQNVDTNVA | 14 |
| L-CDR2 of the OKT10 light chain | SASYRYS | 15 |
| L-CDR3 of the OKT10 light chain | QQYDSYPLT | 16 |
| H-FR4 of OKT10 | WGQGTLVTVSS | 17 |
| heavy chain frame region | | |
| H-FR4 of OKT10 light chain frame region | FGQGTKVEIK | 18 |
| OKT10 heavy chain variable region | EVKLQESGGGLVQPGGSLKLSCAASGFDFSRSWMNWVRQAPGKGLEW IGEINPDSSTINYTTSLKDKFIISRDNAKNTLYLQMTKVRSEDTALYYCA RYGNWFPYWGQGTLVTVSA | 19 |
| OKT10 light chain variable region | DIVMTQSPKIMPTSVGDRVSVTCKASQNVDTNVAWYQQKPGQSPKALI YSASYRYSGVPDRFTGSGSGTDFTLTITNVQSEDLAEYFCQQYDSYPLTF GAGTKLDLK | 20 |
| OKT10 humanized heavy chain variable region | EVQLVESGGGLVQPGGSLRLSCAASGFDFSRSWMNWVRQAPGKGLEW VSEINPDSSTINYTTSLKDRFTISRDNAKNSLYLQMNSLRAEDTAVYYCA RYGNWFPYWGQGTLVTVSS | 21 |
| OKT10 humanized light chain variable region | DIQMTQSPSSLSASVGDRVTITCKASQNVDTNVAWYQQKPGKAPKALI YSASYRYSGVPSRFSGSGSGTDFTLTISSLQPEDFATYYCQQYDSYPLTF [?]GQGTKVEIK | 22 |
| OKT10 Humanized Heavy Chain | EVQLVESGGGLVQPGGSLRLSCAASGFDFSRSWMNWVRQAPGKGLEW VSEINPDSSTINYTTSLKDRFTISRDNAKNSLYLQMNSLRAEDTAVYYCA RYGNWFPYWGQGTLVTVSSASTKGPSVFPLAPSSKSTSGGTAALGCLV KDYFPEPVTVSWNSGALTSGVHTFPAVLQSSGLYSLSSVVTVPSSSLGT QTYICNVNHKPSNTKVDKKVEPKSCDKTHTCPPCPAPELLGGPSVFLFPP KPKDTLMISRTPEVTCVVVDVSHEDPEVKFNWYVDGVEVHNAKTKPRE EQYNSTYRVVSVLTVLHQDWLNGKEYKCKVSNKALPAPIEKTISKAKG QPREPQVYTLPPSREEMTKNQVSLTCLVKGFYPSDIAVEWESNGQPENN YKTTPPVLDSDGSFFLYSKLTVDKSRWQQGNVFSCSVMHEALHNHYTQ KSLSLSPGK | 23 |
| OKT10 Humanized Light Chain | DIQMTQSPSSLSASVGDRVTITCKASQNVDTNVAWYQQKPGKAPKALI YSASYRYSGVPSRFSGSGSGTDFTLTISSLQPEDFATYYCQQYDSYPLTF GQGTKVEIKRTVAAPSVFIFPPSDEQLKSGTASVVCLLNNFYPREAKVQ WKVDNALQSGNSQESVTEQDSKDSTYSLSSTLTLSKADYEKHKVYACE VTHQGLSSPVTKSFNRGEC | 24 |
| OKT10-Hu-IgG1-Pep-C S heavy chain | EVQLVESGGGLVQPGGSLRLSCAASGFDFSRSWMNWVRQAPGKGLEW VSEINPDSSTINYTTSLKDRFTISRDNAKNSLYLQMNSLRAEDTAVYYCA RYGNWFPYWGQGTLVTVSSASTKGPSVFPLAPSSKSTSGGTAALGCLV KDYFPEPVTVSWNSGALTSGVHTFPAVLQSSGLYSLSSVVTVPSSSLGT QTYICNVNHKPSNTKVDKKVEPKSCDKTHTCPPCPAPELLGGPSVFLFPP KPKDTLMISRTPEVTCVVVDVSHEDPEVKFNWYVDGVEVHNAKTKPRE EQYNSTYRVVSVLTVLHQDWLNGKEYKCKVSNKALPAPIEKTISKAKG QPREPQVYTLPPSREEMTKNQVSLTCLVKGFYPSDIAVEWESNGQPENN YKTTPPVLDSDGSFFLYSKLTVDKSRWQQGNVFSCSVMHEALHNHYTQ KSLSLSPGKPTLYNVSLVMSDTAGTSY | 25 |
| Daratumuma b heavy chain variable region | | 26 |
| Daratumuma b light chain variable region | | 27 |
| Isatuximab heavy chain variable region | | 28 |
| Isatuximab heavy chain variable region | | 29 |
| Cynomopho be monkey CD38 extracellular end | LPRWRQQWSGSGTTSRFPETVLARCVKYTEVHPEMRHVDCQSVWDAF KGAFISKYPCNITEEDYQPLVKLGTQTVPCNKTLLWSRIKDLAHQFTQV QRDMFTLEDMLLGYLADDLTWCGEFNTFEINYQSCPDWRKDCSNNPVS VFWKTVSRRFAETACGVVHVMLNGSRSKIFDKNSTFGSVEVHNLQPEK VQALEAWVIHGGREDSRDLCQDPTIKELESIISKRNIRFFCKNIYRPDKFL QCVKNPEDSSCLSGI | 30 |
| Human IgG1-Pep-C S | ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKKV EPKSCDKTHTCPPCPAPELLGGPSVFLFPPKPKDTLMISRTPEVTCVVVD VSHEDPEVKFNWYVDGVEVHNAKTKPREEQYNSTYRVVSVLTVLHQD WLNGKEYKCKVSNKALPAPIEKTISKAKGQPREPQVYTLPPSREEMTKN QVSLTCLVKGFYPSDIAVEWESNGQPENNYKTTPPVLDSDGSFFLYSKL TVDKSRWQQGNVFSCSVMHEALHNHYTQKSLSLSPGKPTLYNVSLVMS DTAGTSY | 31 |
| IgG1-L309X | ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKKV EPKSCDKTHTCPPCPAPELLGGPSVFLFPPKPKDTLMISRTPEVTCVVVD VSHEDPEVKFNWYVDGVEVHNAKTKPREEQYNSTYRVVSVLTVXHQD WLNGKEYKCKVSNKALPAPIEKTISKAKGQPREPQVYTLPPSREEMTKN QVSLTCLVKGFYPSDIAVEWESNGQPENNYKTTPPVLDSDGSFFLYSKL TVDKSRWQQGNVFSCSVMHEALHNHYTQKSLSLSPGK* | 32 |
| 50G12-Hu-I gG1-L309W -E345R | QVQLVQSGAEVKKPGASVKVSCKASGYTFNTYWMQWVRQAPGQG LEWMGAIYPGDGDITYNQKFKGRVTLTADKSTSTVYMELSSLRSED TAVYYCAREGYYYGGALDYWGQGTLVTVSSASTKGPSVFPLAPSSKS TSGGTAALGCLVKDYFPEPVTVSWNSGALTSGVHTFPAVLQSSGLYSLS SVVTVPSSSLGTQTYICNVNHKPSNTKVDKKVEPKSCDKTHTCPPCPAP ELLGGPSVFLFPPKPKDTLMISRTPEVTCVVVDVSHEDPEVKFNWYVDG VEVHNAKTKPREEQYNSTYRVVSVLTVWHQDWLNGKEYKCKVSNKA LPAPIEKTISKAKGQPRRPQVYTLPPSREEMTKNQVSLTCLVKGFYPSDI AVEWESNGQPENNYKTTPPVLDSDGSFFLYSKLTVDKSRWQQGNVFSC SVMHEALHNHYTQKSLSLSPGK | 33 |
| IgG1-L309 W-E345R | ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKKV EPKSCDKTHTCPPCPAPELLGGPSVFLFPPKPKDTLMISRTPEVTCVVVD VSHEDPEVKFNWYVDGVEVHNAKTKPREEQYNSTYRVVSVLTVWHQD WLNGKEYKCKVSNKALPAPIEKTISKAKGQPRRPQVYTLPPSREEMTKN QVSLTCLVKGFYPSDIAVEWESNGQPENNYKTTPPVLDSDGSFFLYSKL TVDKSRWQQGNVFSCSVMHEALHNHYTQKSLSLSPGK | 34 |
| H-CDR1 of 50G12 heavy chain | TYWMQ | 35 |
| H-CDR2 of 50G12 heavy chain | AIYPGDGDITYNQKFKG | 36 |
| H-CDR3 of 50G12 heavy chain | EGYYYGGALDY | 37 |
| L-CDR1 of the 50G12 light chain | TASSSVSSSYLH | 38 |
| L-CDR2 of the 50G12 light chain | GTSNLAS | 39 |
| L-CDR3 of the 50G12 light chain | HRYHRSPWT | 40 |

| | | |
|---|---|---|
| * Wherein, X=E, F, H, C, D, N, Q, R, S, T, K, W, Y. | | |

### Example 1 Preparation of monoclonal antibody against human CD38 (50G12)

50G12-Humanized (hereinafter referred as 50G12-Hu-IgG1) is a humanized monoclonal antibody against CD38, and its heavy and light chain amino acid sequences are derived from SEQ ID NO: 11 and SEQ ID NO: 12 of CN202010805420.2 (i.e., SEQ ID NO: 5 and 6 in the present invention). Heavy chain variable region and light chain variable region of amino acid sequences of 50G 12-Hu-IgG 1 are shown in SEQ ID NO: 1 and 2. The amino acid sequences of H-CDR1, H-CDR2, and H-CDR3 in the heavy chain of 50G12 Hu IgG1 are as shown in SEQ ID NO: 35, 36, and 37, respectively. The amino acid sequences of L-CDR1, L-CDR2, and L-CDR3 in the light chain of 50G12 Hu IgG1 are as shown in SEQ ID NO: 38, 39, and 40, respectively. The heavy chain constant region of 50G12 Hu IgG1 is human IgG1 (amino acid sequence as SEQ ID NO: 3), and the light chain constant region is human Kappa (amino acid sequence as SEQ ID NO: 4). Herein, the coding genes for the heavy and light chains of 50G 12-Hu-IgG 1 were named as 50G12-Hu-IgG1-HC and 50G12-Hu-IgG1-LC, respectively. The genes of 50G12-Hu-IgG1-HC and 50G12-Hu-IgG1-LC were constructed into pcDNA3.4 expression vectors, and the antibodies were expressed and purified after the combination of the two vectors, and the obtained antibodies were named as 50G 12-Hu-IgG 1.

### Example 2 Modification of Fc segment of constant heavy chain of antibody

According to literature (Rowley T F, Peters S J, Aylott M, et al. Engineering hexavalent Fc proteins with enhanced Fc gamma receptor efficacy provide insights into immune complex interactions [J]. Communications biology, 2018, 1 (1): 1-12), it is known that hexameric antibody complexes will be readily generated after the L309 (Eu numbering scheme) of human IgG1 monoclonal antibodies is mutated into cysteine (abbreviated as C) and the tail-piece of human IgM is fused at the Fc terminal, which indicates that the human IgG1 L309C mutation and tail-piec of human IgM have the potential to promote the formation of hexamers in human IgG1.

### 1. Site-directed mutation at position 309 of Fc segment

Herein, in this example, a site-directed mutation was carried out at the L309 position of the 50G 12-Hu-IgG 1 heavy chain gene, and L309 was mutated into (E/F/H/C/D/N/Q/R/S/T/K/W/Y) respectively, and a series of mutants were produced. And then the antibody was expressed and purified according to the above method, and the obtained antibodies were named 50G12-Hu-IgG1-L309X respectively (X represents E/F/H/C/D/N/Q/R/S/T/K/W/Y).

The method for measuring CDC was described as follows: Raji and Daudi cells were purchased from the American Type Culture Collection (ATCC) and were cultured and passaged according to the recommended methods by ATCC. Human serum (Ausiels; Product number: PB022-C) was added to RPMI-1640 (Gibco; Product number: 11835-030) to achieve a final concentration of 5% in human serum; Logarithmic Raji/Daudi cells on this culture medium were resuspended, then they were inoculated into a 96 well plate (Corning; product number: CLS3599), and 50 µ L of cell suspension (including 100000 cells) was inoculated into each well; The antibody to be tested was diluted in a gradient and was added to the 96 well plate containing the cells mentioned above at 50 µL per well; After mixing, the cells were incubated in a carbon dioxide cell culture incubator for 2.5 hours; CCK-8 (Dojindo, item number: CK04) was added to a 96 well plate at 20 µL per well, and was incubated for another 2 hours; SpectraMax 190 (Molecular Devices) was used to read the OD450 of a 96 well plate; Data were analyzed and plotted using GraphPad Prism7 to calculate IC₅₀.

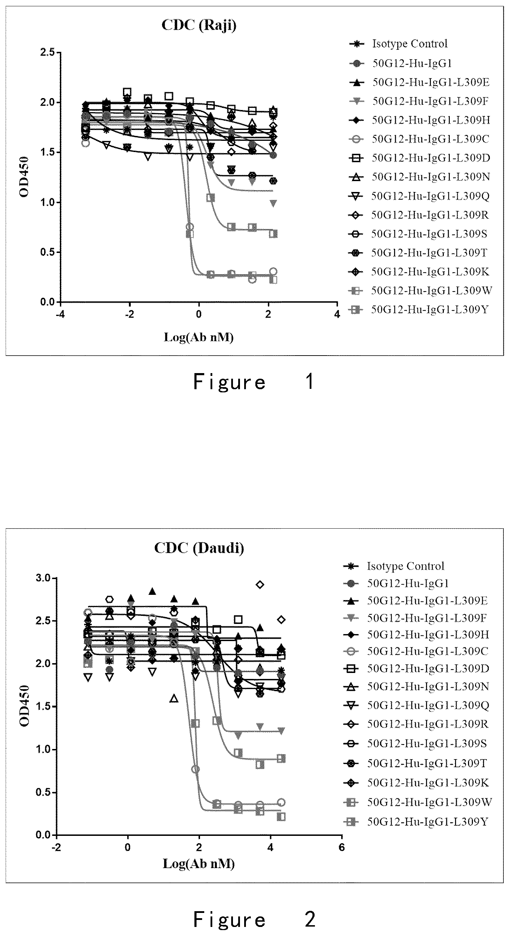

The stronger the CDC activity was, the lower the cell viability and the lower the OD450. Both Figure 1 and Figure 2 show that compared with the maternal antibody 50G 12-Hu-IgG 1, 50G12-Hu-IgG1-L309F/C/W/Y exhibits significantly enhanced CDC. Wherein, isotype Control is a human IgG1 Clone antibody that does not bind to Daudi cells.

### 2. Modification of the terminal of Fc segment

Here, the coding sequence of the tail-piece(Amino acid sequence: PTLYNVSLVMSDTAGTCY (SEQ ID NO: 7), and the short peptide of this segment was abbreviated as Pep) of IgM was connected to the end of the 50G12-Hu-IgG1 heavy chain gene by genetic engineering, and the heavy chain gene was named as 50G12-HU-IGG1-PEP-HC (amino acid sequence shown as SEQ ID NO: 9). To avoid the formation of disulfide bonds between antibody molecules during the expression process, cysteines in the two tail-pieces were mutated into serines (the amino acid sequence of the mutated tail piece was PTLYNVSLVMSDTAGTSY (SEQ ID NO: 8), which was abbreviated as Pep-CS), and the heavy chain gene was named as 50G12-Hu IgG1 Phep-CS-HC (amino acid sequence was SEQ ID NO: 10). The genes of 50G12-Hu-IgG1-HC and 50G12-Hu-IgG1-LC were constructed into pcDNA3.4 expression vectors, and the antibodies were expressed and purified after the combination of the two vectors, and the obtained antibodies were named as 50G12-Hu-IgG1-Pep and 50G12-Hu-IgG1-PEP-CS, respectively.

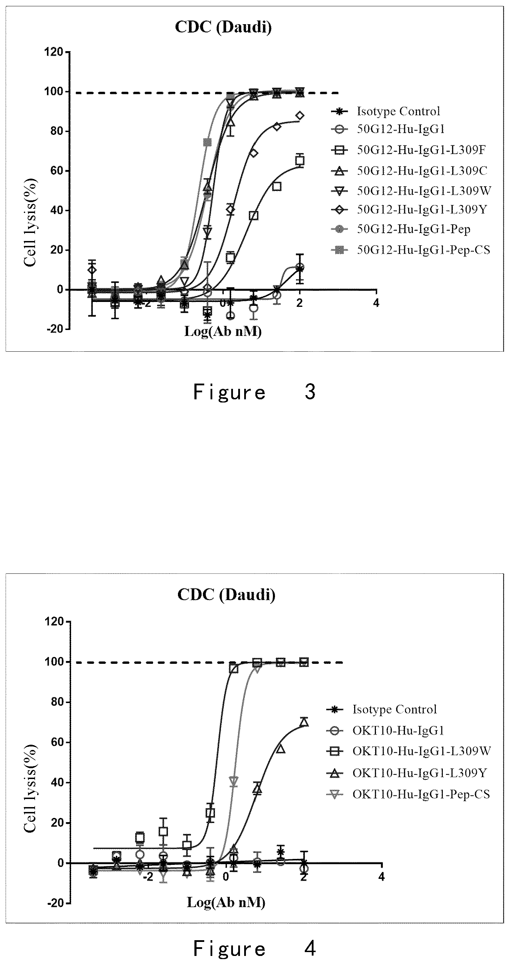

CDC of 50G12-Hu-IgG1-L309F/C/W/Y, 50G12-Hu-IgG1-Pep and 50G12-Hu-IgG1-Pep-CS was detected by the above method with a difference that CellTiter-Glo^{®} Luminescent Cell Viability Assay (Promega, item number G7572, abbreviated as CTG) was used to detect cell viability. Triton X-100 (0.1%) was added to some wells to fully lyse the cells, and the signal generated by adding CTG to this group of cells was the basal value. The signal generated by cells without antibody treatment after adding CTG was the maximum value. CDC was calculated according to the following formula: Cytotoxicity (%)=(Max - experimental value)/(Max - base value)× 100. GraphPad Prism7 was used for mapping and data analysis, as shown in Figure 3. EC50 was calculated as shown in Table 2.

**Table 2 CDC activity of the antibodv of the oresent invention**

| Antibody | Top | EC₅₀ (nM) |
|---|---|---|
| 50G12-Hu-IgG1 | 11.37 | NA |
| 50G12-Hu-IgG1-L309F | 63.65 | 4.087 |
| 50G12-Hu-IgG1-L309C | 99.59 | 0.3727 |
| 50G12-Hu-IgG1-L309W | 99.82 | 0.55 |
| 50G12-Hu-IgG1-L309Y | 85.23 | 1.841 |
| 50G12-Hu-IgG1-Pep | 100.7 | 0.3976 |
| 50G12-Hu-IgG1-Pep-CS | 99.7 | 0.2315 |

The stronger the CDC is, the smaller the EC₅₀, and the higher the Top are (height of the high platform on the curve). Both Figure 3 and Figure 2 show that compared with the maternal antibody 50G12-Hu-IgG1, 50G12-Hu-IgG1-L309F/C/W/Yexhibits significantly enhanced CDC, and the CDC of 50G12-Hu-IgG1-L309F/Y is weaker than that of 50G12-Hu-IgG1-L309C/W. 50G12-Hu-IgG1-Pep and 50G12-Hu-IgG1-Pep-CS have similar CDC, and their CDC are basically comparable to those of 50G12-Hu-IgG1-L309C/W. The Top of 50G12-Hu-IgG1-L309C/W, 50G12-Hu-IgG1-Pep, and 50G12-Hu-IgG1-Pep-CS are all greater than 99, indicating that these antibodies are able to fully lyse target cells at high concentrations.

### Example 3 Aggregate analysis of antibody mutants

The purity of the selected antibody mutants were determined using HPLC-SEC. The results are shown in below:

**Table 3. The preferred purity of the antibody mutant of the present invention**

| Antibody | SEC main peak (%) |
|---|---|
| 50G 12-Hu-IgG1 | 99.3 |
| 50G12-Hu-IgG1-L309F | 99.5 |
| 50G12-Hu-IgG1-L309C | 79.7 |
| 50G12-Hu-IgG1-L309W | 99.4 |
| 50G12-Hu-IgG1-L309Y | 98.6 |
| 50G12-Hu-IgG1-Pep | 74.0 |
| 50G12-Hu-IgG1-Pep-CS | 99.5 |

The determination results of HPLC-SEC show that the proportions of main peaks of 50G12-Hu IgG1-L309F/W/Y and 50G12-Hu IgG1 Phep-CS are both greater than 98%, indicating their small size heterogeneity and high purity. In addition, the proportions of main peaks of 50G12-Hu-IgG1-L309C and 50G12-Hu-IgG1-Pep are 79.7% and 74.0%, respectively, and the purity of both were lower than 80%. The spectrum shows that there are many aggregates in these two antibodies.

### Example 4 Modification of anti-human CD38 monoclonal antibody OKT10

### 1. Preparation of humanized monoclonal antibody OKT10

OKT10 is an anti-human CD38 monoclonal antibody produced by mouse hybridoma. Its heavy chain variable region and light chain variable region amino acid sequences (SEQ ID NOs: 19 and 20) are from NCBI GenBank(ABA42888.1 and ABA42887.1).

The DNA encoding the humanized heavy chain and light chain variable region was synthesized by Shanghai Shengong Bioengineering Co., LTD. The amino acid sequences of the heavy chain variable region and the light chain variable region of OKT10 were analyzed, and the complementary determinant region and the frame region of the heavy chain and the light chain of OKT10 were determined according to the Kabat rule. The amino acid sequences of the heavy chain CDRs of 50G12 are H-CDR1: SEQ ID NO: 11, H-CDR2: SEQ ID NO: 2 and H-CDR3: SEQ ID NO: 13, and the amino acid sequences of the light chain CDRs are L-CDR1: SEQ ID NO: 14, L-CDR2: SEQ ID NO: 5 and L-CDR3: SEQ ID NO: 16.

In the https://www.ncbi.nlm.nih.gov/igblast/, the homology of the murine OKT10 heavy chain variable region was compared with the human IgG germline sequence, IGHV3-48*01 was selected as the heavy chain CDR transplantation template, the murine OKT10 heavy chain CDR was transplanted into the IGHV3-48*01 backbone region, and WGQGTLVTVSS (SEQ ID NO: 17) was added after H-CDR3 as the fourth framework region, so that the variable region sequence of the CDR transplanted heavy chain was obtained. Similarly, the light chain variable region of murine antibody 50G12 was compared with the sequence homology of human IgG germ line. IGKV1-39*01 was selected as the light chain CDR transplantation template, and the light chain CDR of murine antibody 50G12 was transplanted into the skeleton region of IGKV1-39*01. FGQGTKVEIK was added after L-CDR3 as the fourth frame region to obtain the light chain variable region sequence of CDR transplantation. On the basis of CDR transplantation of variable regions, some amino acid sites in the framework region are subjected to reverse mutations (reverse mutations refer to the mutation of certain amino acids in the human framework region into amino acids in the same position in the mouse framework region, and the reverse mutation sites are generally crucial for maintaining the structure and/or affinity of antibodies). During mutation, amino acid sequence was encoded by Kabat, and the location of site was indicated by Kabat code.

Preferably, for the heavy chain variable region of CDR transplantation, the T at position 28 was reverted to D. For CDR transplantation of light chain variable regions, F at position 36 was mutated to Y, and S at position 46 was mutated to A.

The heavy chain variable and light chain variable with revert mutation sites were defined as the heavy chain variable (amino acid sequence such as SEQ ID NO: 21) and light chain variable (amino acid sequence such as SEQ ID NO: 22) of humanized OKT10, respectively. The DNA encoding the humanized heavy chain and light chain variable region was synthesized by Shanghai Shengong Bioengineering Co., LTD. The synthetic humanized heavy chain variable region was linked to the constant region of human IgG1 (amino acid sequence shown as SEQ ID NO: 3), so as to obtain the humanized heavy chain gene in full-length which was named as OKT10-Hu-HC (amino acid sequence shown as SEQ ID NO: 23). By connecting the variable region of the human light chain with the constant region of the human Kappa chain (amino acid sequence such as SEQ ID NO: 4), a human light chain gene in full-length was obtained and named as OKT10-Hu-LC (amino acid sequence shown as SEQ ID NO: 24).

The genes of OKT10-Hu-HC and OKT10-Hu-LC were constructed into pcDNA3.4 expression vectors, and the antibodies were expressed and purified after the combination of the two vectors, and the obtained antibodies were named as OKT10-Hu-IgG1.

### 2. Preparation of OKT10-Hu-IgGl mutant

Herein, site directed mutagenesis was performed on the L309 position of theOKT10-Hu-IgG1 heavy chain gene, which was mutated into W or Y, respectively. The mutated genes were then combined with OKT10-Hu-LC to express and purify the antibodies. The resulting antibodies were named as OKT10-Hu-IgG1-L309W and OKT10-Hu IgG1-L309Y, respectively. Herein, the coding sequence of Pep-CS was connected to the terminal of the OKT10-Hu-IgG1 heavy chain gene through genetic engineering, and the heavy chain gene is named as OKT10-Hu-IgG1-Pep-CS-HC (amino acid sequence shown as SEQ ID NO: 25). After combining with OKT10-Hu-LC, the antibody was expressed and purified according to the above method, and the resulting antibody was named as OKT10-Hu-IgG1-Pep-CS.

### 3 Determination of CDC of OKT10-Hu-IgG1 mutant

The CDC of OKT10 Hu IgG1 and its mutants were measured using the method described in the above example. The result was shown in Figure 4.

The results in Figure 4 show that OKT10-Hu-IgG1-L309W, OKT10-Hu-IgG1-L309Y, and OKT10-Hu-IgG1-Pep-CS have significantly enhanced CDC activity compared to the parent antibody OKT10-Hu-IgG1, with EC50 of 0.5942 nM, 5.902 nM, and 1.715 nM, respectively, and their high plateaus of 99.85, 69.84, and 99.43, respectively. The Top of-OKT10/W, 50G12-Hu-IgG1-Pep, and 50G12-Hu-IgG1-Pep-CS are all greater than 99, indicating that these antibodies are able to fully lyse target cells at high concentrations. The above results indicate that among the three, OKT10-Hu IgG1-L309W has the strongest CDC.

### 4. Determination of the binding ability of OKT10-Hu-IgG1 to cynomolgus monkey CD38

Daratumumab is an approved anti-human CD38 monoclonal antibody with amino acid sequences in the variable regions of heavy and light chains (SEQ IDs NOs: 26 and 27) from WHO Drug Information, Vol. 24, No. 1, 2010.

Isatuximab is another approved monoclonal antibody against human CD38, with its heavy and light chain variable amino acid sequences (SEQ ID NOS: 28 and 29) from WHO Drug Information, Vol. 29, No. 3, 2015.

The above-mentioned heavy and light chain variable region DNAs were synthesized by Shanghai Shenggong Bioengineering Co., Ltd. A heavy chain gene in full-length was obtained by linking the synthetic Daratumumab heavy chain variable region gene with the human IgG1 heavy chain constant region gene, and the light chain gene in full-length was obtained by linking the Daratumumab light chain variable region gene with the human Kappa chain constant region gene. The antibody was expressed and purified using the method described in the above embodiment, and the resulting antibody was named as Daratumumab-IgG1 (also known as Daratumumab). In addition, antibodies Isatuximab-IgG1 were obtained with similar experimental methods.

Amino acid sequence of cynomolgus monkey CD38 (SEQ ID NO: 30) was from https://www.uniprot.org/uniprot/Q5VAN0. The DNA encoding the extracellular segment of cynomolgus monkey CD38 was synthesized by Shanghai Sanggong Bioengineering Co., Ltd. , and the coding sequence encoding polyhistidine was added to the end of the gene, and then the recombinant gene was constructed into an expression vector. The recombinant protein was expressed by the method described in the above embodiment, and then the recombinant protein in the culture supernatant was purified by a Ni-NTA affinity chromatography column, and the resulting recombinant protein was named as CD38-ECD-Cyno.

Microplate (10 ng/well) was coated with CD38-ECD-Cyno, and the binding ability of CD38-ECD-Cyno to 50G 12-Hu-IgG 1, OKT10-Hu-IgG1, Daratumumab-IgG1, and Isatuximab-IgG1 was determined by ELISA.

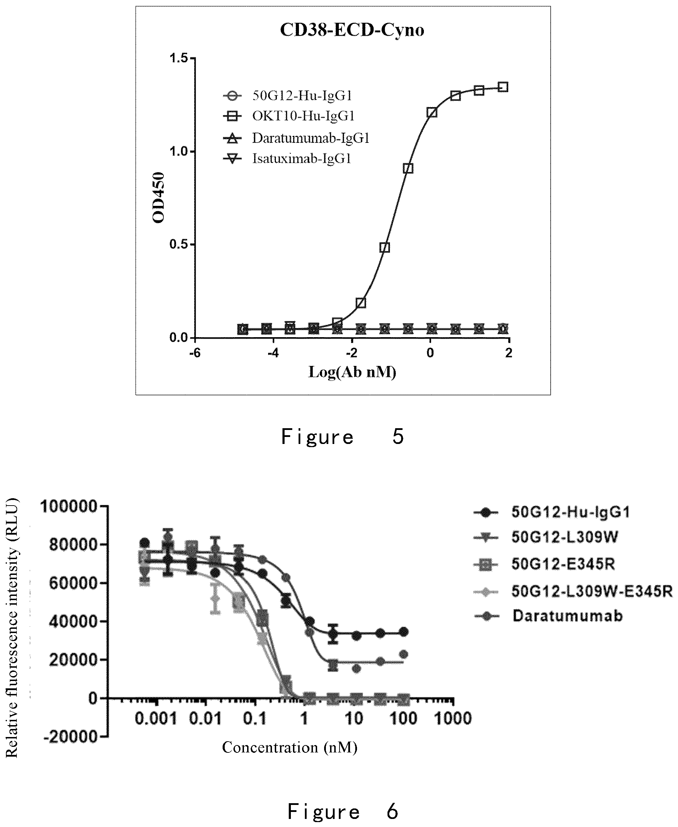

Figure 5 shows that OKT10-Hu-IgG1 can effectively bind to cynomolgus monkeys CD38, with an EC₅₀ of 0.1324 nM. 50G12 Hu IgG1, Daratumumab IgG1, and Isatuximab IgG1 cannot recognize cynomolgus monkeys CD38.

### Example 5 Preparation of monoclonal antibody 50G12-L309W-E345R

By genetic engineering and molecular cloning techniques, L309 in the constant heavy chain region of 50G 12-Hu-IgG 1 was site-directedly mutated to W, and E345 to R was site-directedly mutated to R, and the resulting heavy chain was named as 50G12-hu-IgG1-L309W-E345R in pcDNA3.4 expression vector. The above heavy chain was co-transfected with 50G12-Hu-IgG1-LC into HEK-293F cells for expression for 5 days. The antibody obtained by ProteinA affinity chromatography was named as 50G12-L309W-E345R. The same method was used to obtain single point mutant antibodies 50G12-L309W (also known as 50G12-Hu-IgG1-L309W) and 50G12-E345R.

### Example 6: Determination of affinity of 50G12-L309W-E345R to human CD38

Herein, the binding and dissociation constants of 50G12-L309W-E345R, 50G12-Hu-IgG1, and Daratumumab to human CD38 protein were determined by Biacore 8K (purchased from GE Healthcare). The specific Example methods were as follows:
1) Human CD38 protein was biotinized according to the EZ-Link NHS-Biotin Reagent manual (purchased from Thermo Fisher Scientific, Cat. No. 21343) and named as CD38-biotin;
2) CAP chip conjugation: 50ug/mL Biotin CAPture Reagent was mixed at 1:1 with HBS-EP+ pH7.4 Buffer at a contact time of 60 seconds and a flow rate of 2 µL/min.
3) Biotin-CD38 was captured with the following running parameters: The concentration of biotin-CD38 was 2 µg/mL, the contact time was 15 s, the flow rate was 10 µL/min, and the regeneration contact time was 30 s.
4) Each antibody to be tested was diluted with HBS-EP+ pH7.4 buffer to a maximum concentration of 200 nM, diluted to 1.5625 nM and 0 concentration points by 2-fold, the regeneration solution was prepared according to the Biotin CAPture Kit instructions, and was injected on Biacore 8K according to the following parameters: binding time 180s, dissociation time 600s, flow rate 30 µL/min, regeneration contact time 30 s, flow rate 30 µL/min.
5) Data were analyzed by using Biocore 8K Evaluation Software, the "1: 1 binding kinetics model" formula was selected for fitting in the "Kinetics" mode, so as to obtain the affinity data between each antibody and CD38.

As shown in Table 4, the affinities of 50G12-L309W-E345R and 50G 12-Hu-IgG 1 to CD38 protein were stronger than that of Daratumumab, which showed that the dissociation constants kd of 50G12-L309W-E345R and 50G 12-Hu-IgG 1 were better than those of Daratumumab, and the equilibrium dissociation constants KD of the three antibodies were 2.99×10⁻⁹ M, 3.58×10⁻⁹ M and 2.60×10⁻⁸ M, respectively.

**Table 4. Binding affinity of 50G12-L309W-E345R to human CD38 protein**

| **Sample** | **ka (1/Ms)** | **kd (1/s)** | **KD (M)** |
|---|---|---|---|
| 50G12-Hu-IgG1 | 1.71E+05 | 6.13E-04 | 3.58E-09 |
| 50G12-L309W-E345R | 3.46E+05 | 1.03E-03 | 2.99E-09 |
| Daratumumab | 1.30E+05 | 3.37E-03 | 2.60E-08 |

### Example 7 CDC activity assay for 50G12-L309W-E345R

Human Burkitt's lymphoma cells Daudi, Raji and Romas in the logarithmic growth phase were washed with DPBS once, the cell density was adjusted to 2E5/mL in serum-free phenol-free red 1640 medium, and the white 96-well cell culture plates were plated with the cells at 50 µL/well; the antibody was diluted to the desired concentration in serum-free phenol red 1640 medium, and then 12 concentrations were serial diluted according to a 3-fold gradient, and the diluted drug was added to the above cell culture plates at 50 µL/ wells so as to get the final concentration of antibody at 50 nM in duplicate, and the plate was incubated at 37°C, 5% CO₂ cell culture incubator for 30 min. Normal Human Serum complement was added to the above culture plate at 20 µL/well, incubated at 37 °C, 5% CO₂ cell cell culture incubator for 2 h, the above cell culture plate was placed at room temperature for equilibration for 15 min, and Cell Titer-Glo was added at room temperature equilibration at 80 µL/well Luminescent Assay chromogen incubated at room temperature for 8 minutes. Data were collected by detection by microplate reader i3 (purchased from Molecular Devices, model SPECTRA MAX i3), data were analyzed and plotted with GraphPad Prism9, and IC₅₀ was calculated.

As shown in Figure 6, the 50G12-L309W-E345R-mediated complement activation exhibits significantly better killing activity on Romas cells than the unmutated antibody 50G 12-Hu-IgG 1 and the control antibody Daratumumab, and slightly better than the single-point mutant antibody 50G12-L309W and the single-point mutant antibody 50G12-E345R. As shown in Table 5, the IC₅₀ of complement effect mediated by 50G12-L309W-E345R on Roma cell killing is about 8 times better than that of Daratumumab; The absolute value of maximum lethal activity (Bottom) is better than Daratumumab, about 98 times; IC₅₀ is better than 50G12-L309W, about 1.7 times; and is better than 50G12-E345R by approximately 1.2 times.

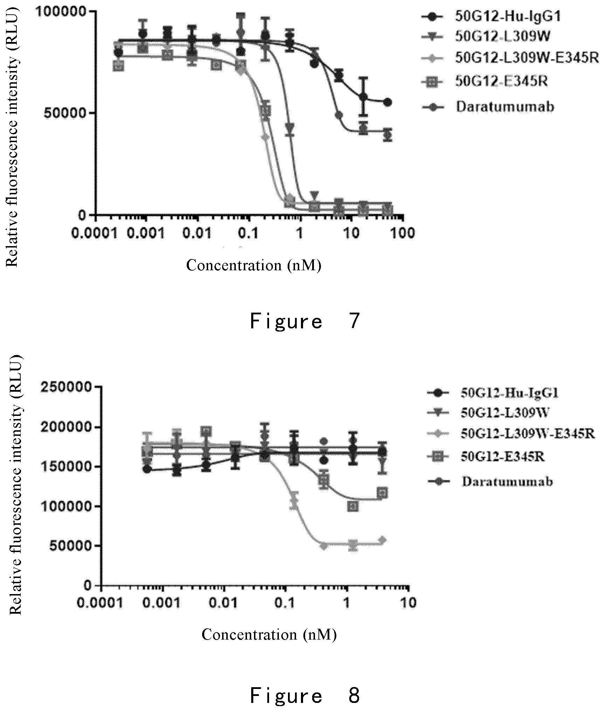

As shown in Figure 7, complement activation mediated by 50G12-L309W-E345R exhibits significantly better killing activity on Daudi cells than single point mutant antibody 50G12-L309W, unmutated antibody 50G12-Hu IgG1, and control antibody Daratumumab, and slightly better than single point mutant antibody 50G12-E345R. As shown in Table 6, the IC₅₀ of complement effect mediated by 50G12-L309W-E345R on Daudi cell killing is about 19 times better than that of Daratumumab; The absolute value of maximum lethal activity (Bottom) is better than Daratumumab, about 25 times; IC50 is better than 50G12-L309W, about 3.2 times; IC50 is better than 50G12-E345R, about 1.4 times.

As shown in Figure 8, the 50G12-L309W-E345R-mediated complement activation exhibits significantly better killing activity on Raji cells than the single-point mutant antibody 50G12-L309W, the single-point mutant antibody 50G12-E345R, the unmutated antibody 50G 12-Hu-IgG 1, the control antibody Daratumumab, and 50G 12-Hu-IgG 1 and Daratumumab showed little CDC activity on Raji cells, which may be related to the relatively low expression level of CD38 on the surface of Raji cells. As shown in Table 7, the IC₅₀ of complement effect mediated by 50G12-L309W-E345R on Raji cell killing is about 2 times better than that of 50G12-E345R; the absolute value of maximum lethal activity (Bottom) is better than 50G12-E345R, about 2.4 times.

The above data indicate that the CDC effects induced by various antibodies vary on different cells, which is not only related to the expression levels of CD38 in different cells, but also to the expression levels of complement regulatory proteins CD55 and CD59 on each cell (reference: CD38 expression and completion inhibitors effect response and resistance to daratumab therapy in myoma [J]. Blood, 2016, 128 (7): 959-970.); But one thing can be certain is that introducing L309W and E345R double point mutations simultaneously in the constant region of the antibody's heavy chain can effectively enhance the antibody's mediated CDC activity, and the introduction of these double point mutations can endow the antibody with unexpectedly excellent activity, which is superior to any antibody that only introduces L309W or E345R single point mutations or does not introduce mutation points. Moreover, the enhancement effect of CDC is more significant in tumor cells with relatively low levels of CD38 expression.

**Table 5. Killing activity of complement activation mediated by 50G12-L309W-E345R on Romas cells**

| **Sample** | **IC₅₀ (nM)** | **SD** | **Bottom** |
|---|---|---|---|
| 50G12-Hu-IgG1 | 0.459 | 0.162 | 33966 |
| 50G12-L309W | 0.162 | 0.014 | 713.6 |
| 50G12- E345R | 0.113 | 0.011 | 192.1 |
| 50G12-L309W-E345R | 0.094 | 0.013 | 192.1 |
| Daratumumab | 0.755 | 0.002 | 18914 |

**Table 6. Killing activity of 50G12-L309W-E345R-mediated complement activation on Daudi cells**

| **Sample** | **IC₅₀ (nM)** | **SD** | **Bottom** |
|---|---|---|---|
| 50G 12-Hu-IgG1 | 4.217 | 0.486 | 51424 |
| 50G12-L309W | 0.575 | 0.048 | 1431 |
| 50G12-E345R | 0.253 | 0.014 | -1777 |
| 50G12-L309W-E345R | 0.177 | 0.006 | 1486 |
| Daratumumab | 3.308 | 0.001 | 36749 |

**Table 7. Killing activity of complement activation mediated by 50G12-L309W-E345R on Raji cells**

| **Sample** | **IC₅₀(nM)** | **SD** | **Bottom** |
|---|---|---|---|
| 50G 12-Hu-IgG1 | NA | NA | 167616 |
| 50G12-L309W | NA | NA | 147519 |
| 50G12-E345R | 0.268 | 0.095 | 104778 |
| 50G12-L309W-E345R | 0.129 | 0.007 | 42978 |
| Daratumumab | NA | NA | 173564 |

### Example 8 Determination of ADCC activity of 50G12-L309W-E345R

Human Burkitt's lymphoma cells Daudi, Ramos, Raji, human myeloma cells NCI-H929, and human multiple myeloma cells MOLP8 in the logarithmic growth phase were washed with DPBS once, and then the cell density was adjusted to 4.8E5/mL serum-free 1640 medium, and spread onto white 96-well cell culture plates at 25 µL/well, each antibody was diluted to the desired concentration with serum-free 1640 medium, and then 10 concentrations were serial diluted according to a 3-fold gradient, and the diluted antibodies were added to the above cell culture plate at 25 µL/well and incubated in a cell culture incubator for 45 minutes, the ADCC effector cells in the logarithmic growth phase were washed with DPBS once, and then the cell density was adjusted to 3E6/mL in serum-free 1640 medium, and the cell density was adjusted to 25 µL/well, add to the above culture plates, and incubate at 37 °C, 5% CO₂ cell culture incubator for 6 h;

The cell culture plate was placed at room temperature for equilibration for about 15 min in advance, 60 µL of Bio-glo chromogenic agent was added to each well, and after 4 min of incubation at room temperature, the data were collected by microplate reader i3, and the data were analyzed and graphed with GraphPad Prism9 and the IC₅₀ was calculated.

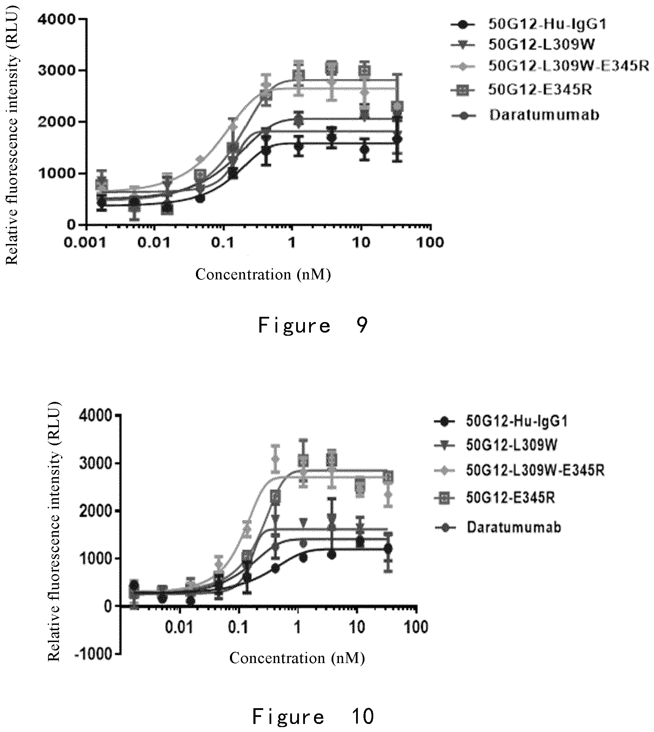

As shown in Figure 9, the ADCC effect mediated by 50G12-L309W-E345R exhibits significantly better killing activity on Daudi cells than the unmodified antibody 50G12-Hu IgG1, single point mutant antibody 50G12-L309W, single point mutant antibody 50G12-E345R, and control antibody Daratumumab. As shown in Table 8, the IC₅₀ of 50G12-L309W-E345R-mediated ADCC effect on Daudi cell killing was about 1.5-fold better than that of Daratumumab, the absolute value of maximum killing activity (Top) was about 1.3-fold better than that of Daratumumab, the IC₅₀ was better than that of 50G12-E345R, about 2-fold, and the IC₅₀ was better than that of 50G12-L309W, about 1.7-fold.

**Table 8 Killing activity of ADCC mediated bv 50G12-L309W-E345R on Daudi cells**

| **Sample** | **IC₅₀ (nM)** | **SD** | **Top** |
|---|---|---|---|
| 50G 12-Hu-IgG1 | 0.219 | 0.114 | 1583 |
| 50G12-L309W | 0.142 | 0.019 | 1818 |
| 50G12-E345R | 0.166 | 0.089 | 2817 |
| 50G12-L309W-E345R | 0.085 | 0.019 | 2653 |
| Daratumumab | 0.132 | 0.034 | 2062 |

As shown in Figure 10, the ADCC effect mediated by 50G12-L309W-E345R was significantly better than that of the unmutated antibody 50G12-Hu-IgG, the single-point mutation antibody 50G12-L309W, the single-point mutation antibody 50G12-E345R, and the control antibody Daratumumab. As shown in Table 9, the IC₅₀ of the ADCC effect mediated by 50G12-L309W-E345R on NCI-H929 cells is about 1.5 times higher than that of Daratumumab; The absolute value of maximum lethal activity (Top) is better than Daratumumab, about 1.9 times; And IC₅₀ is better than 50G12-E345R, about 1.9 times; IC₅₀ is better than 50G12-L309W, about 1.3 times.

**Table 9. Killing activity of ADCC mediated by 50G12-L309W-E345R on NCI-H929 cells**

| **Sample** | **IC₅₀ (nM)** | **SD** | **Top** |
|---|---|---|---|
| 50G12-Hu-IgG1 | 0.298 | 0.075 | 1195 |
| 50G12-L309W | 0.154 | 0.014 | 1614 |
| 50G12-E345R | 0.218 | 0.017 | 2850 |
| 50G12-L309W-E345R | 0.115 | 0.034 | 2711 |
| Daratumumab | 0.172 | 0.125 | 1407 |

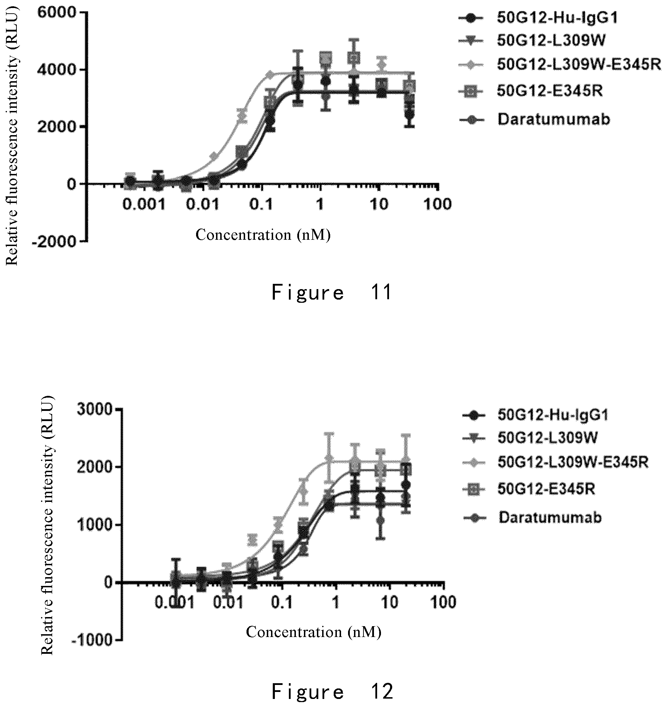

As shown in Figure 11, the ADCC effect mediated by 50G12-L309W-E345R exhibits significantly better killing activity on Romas cells than the unmodified antibody 50G12-Hu IgG1, single point mutant antibody 50G12-L309W, single point mutant antibody 50G12-E345R, and control antibody Daratumumab. As shown in Table 10, the IC₅₀ of 50G12-L309W-E345R-mediated ADCC effect on Romas cell killing is about 2.7 times better than that of daratumumab, the absolute maximum killing activity (Top) is about 1.2 times better than that of Daratumumab, the IC50 is better than that of 50G12-E345R, about 2.4 times, and the IC50 is better than that of 50G12-L309W, about 2 times.

**Table 10. Killing activity of ADCC mediated by 50G12-L309W-E345R on Romas cells**

| Sample | **IC₅₀(nM)** | **SD** | **Top** |
|---|---|---|---|
| 50G12-Hu-IgG1 | 0.095 | 0.019 | 3193 |
| 50G12-L309W | 0.070 | 0.006 | 3244 |
| 50G12-E345R | 0.080 | 0.005 | 3894 |
| 50G12-L309W-E345R | 0.034 | 0.005 | 3868 |
| Daratumumab | 0.092 | 0.005 | 3201 |

As shown in Figure 12, the ADCC effect mediated by 50G12-L309W-E345R exhibits significantly better killing activity on MOLP8 cells than the unmodified antibody 50G12-Hu IgG1, single point mutant antibody 50G12-L309W, single point mutant antibody 50G12-E345R, and control antibody Daratumumab. As shown in Table 11, the IC₅₀ of ADCC mediated by 50G12-L309W-E345R on MOLP8 cell killing is about 3.5 times better than that of Daratumumab; The absolute value of maximum lethal activity (Top) is better than Daratumumab, about 1.5 times; And IC₅₀ is better than 50G12-E345R, about 3.3 times; IC₅₀ is better than 50G12-L309W, about 2.5 times.

**Table 11. Killing activity of ADCC mediated by 50G12-L309W-E345R on MOLP8 cells**

| **Sample** | **IC₅₀ (nM)** | **SD** | **Top** |
|---|---|---|---|
| 50G12-Hu-IgG1 | 0.253 | 0.148 | 1583 |
| 50G12-L309W | 0.197 | 0.021 | 1367 |
| 50G12-E345R | 0.263 | 0.067 | 1953 |
| 50G12-L309W-E345R | 0.079 | 0.017 | 2100 |
| Daratumumab | 0.281 | 0.037 | 1355 |

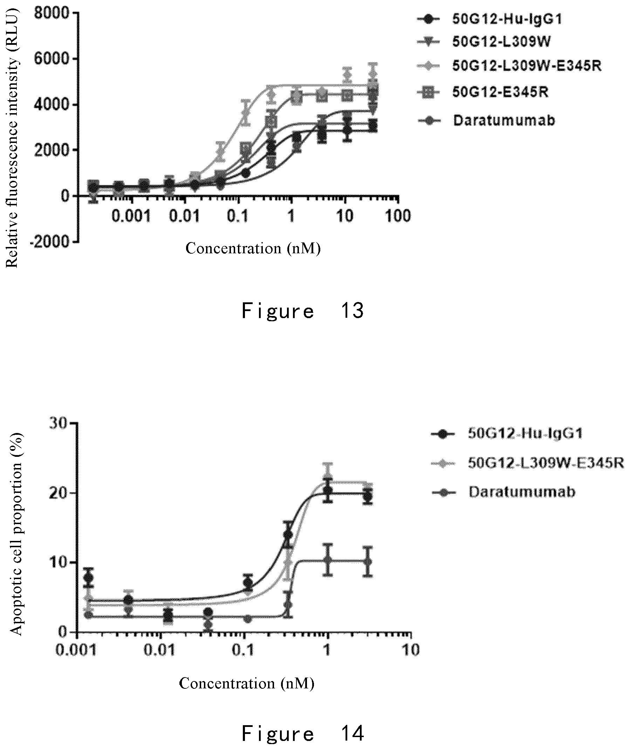

As shown in Figure 13, the ADCC effect mediated by 50G12-L309W-E345R exhibits significantly better killing activity on Raji cells than the unmodified antibody 50G12-Hu IgG1, single point mutant antibody 50G12-L309W, single point mutant antibody 50G12-E345R, and control antibody Daratumumab. As shown in Table 12, the IC₅₀ of ADCC mediated by 50G12-L309W-E345R on Raji cell killing is about 9.2 times better than that of Daratumumab; The absolute value of maximum lethal activity (Top) is better than Daratumumab, about 1.3 times; And IC₅₀ is better than 50G12-E345R, about 2.7 times; IC₅₀ is better than 50G12-L309W, about three times.

**Table 12. Killing activity of ADCC mediated by 50G12-L309W-E345R on Raji cells**

| **Sample** | **IC₅₀ (nM)** | **SD** | **Top** |
|---|---|---|---|
| 50G12-Hu-IgG1 | 0.279 | 0.063 | 2855 |
| 50G12-L309W | 0.229 | 0.072 | 3175 |
| 50G12-E345R | 0.200 | 0.020 | 4455 |
| 50G12-L309W-E345R | 0.075 | 0.014 | 4850 |
| Daratumumab | 0.690 | 0.167 | 3725 |

The above data indicate that the simultaneous introduction of L309W and E345R two-point mutations in the constant region of the antibody heavy chain can effectively enhance the ADCC activity mediated by the antibody. Its activity is superior to any antibody onto which only L309W or E345R single point mutation is introduced or no mutations are introduced, and for the cell, NCI-H929 (Reference: CD38 expression and complement inhibitors affect response and resistance to daratumumab therapy in myeloma [J]. Blood, 2016, 128(7):959-970.) which is not sensitive to the CDC effect, L309W and E345R two-point mutations can still exhibit better ADCC enhancement effects.

### Example 9 Apoptosis induced by 50G12-L309W-E345R

Human Burkitt's lymphoma cells Daudi at logarithmic growth stage were washed once with RPMI-1640 culture medium containing 2%FBS, and the cell density was adjusted according to 1E5/150µL/well, and then spread on a 96-well cell culture plate. The antibody was diluted to 12nM in RPMI-1640 medium containing 2% FBS, and then the diluted antibody was added to the 96-well cell culture plate according to a triple gradient for 8 consecutive concentrations at 50µL/well. The final concentration of antibody was 3nM, and the antibody was incubated in the cell incubator for 24h. The cells were washed twice with precooled PBS at 200µL/well, 400g, centrifuged at 4°C for 5min. Annexin V Annexin V apoptosis detection kit labeled by FITC was used to stain the apoptotic cells, i.e. 100µL 1×binding buffer suspension cells, 1.5µL/ well annexin V FITC-Annexin V were added, gently mixed and reacted at room temperature without light for 15min. The 100µL 1×binding buffer was added, mixed, and the average fluorescence intensity of the FITC channel was determined by flow cytometry (purchased from Beckman, cytoflex) within 1h, and the data were analyzed to calculate the number of stained cells. GraphPad Prism9 was used for data analysis and mapping, and IC₅₀ was calculated.

As shown in Figure 14, the apoptotic effect of 50G12-L309W-E345R on Daudi cells is comparable to that of the unmodified antibody 50G12-Hu IgG1, both of which are significantly better than the control antibody Daratumumab. As shown in Table 13, the maximum activity (Top) of 50G12-L309W-E345R induced apoptosis in Daudi cells is about 2.1 times higher than that of Daratumumab.

**Table 13.Aoootosis of Daudi cells induced bv 50G12-L309W-E345R**

| **Sample** | **IC₅₀ (nM)** | **SD** | **Top** |
|---|---|---|---|
| 50G12-Hu-IgG1 | 0.270 | 0.055 | 19.98 |
| 50G12-L309W-E345R | 0.382 | 0.055 | 21.62 |
| Daratumumab | 0.368 | 0.022 | 10.3 |

All publications mentioned herein are incorporated by reference as if each individual document was cited as a reference in the present application. It should be understood that, after reading the above teachings of the present invention, those skilled in the art can make various modifications and changes. These equivalent forms are also within the scope defined by the claims appended hereto.

## Claims

1. An antibody or fragment or fusion protein thereof with antigen-binding activity and CDC activity, which comprises a binding functional domain targeting a predetermined antigen, and a heavy chain Fc region element, wherein the heavy chain Fc region element comprises:
(1) an amino acid residue at position 309 in the Fc region selected from the group consisting of: W, Y, E, F, H, C, D, N, Q, R, S, T, and K; and/or
(2) a tail-piece element at the C-terminus.

2. The antibody or fragment or fusion protein thereof of claim 1, wherein the antibody or fragment or fusion protein thereof further has ADCC activity.

3. The antibody or fragment or fusion protein thereof of any one of claims 1-2, wherein the heavy chain Fc region element comprises amino acid residue substitutions at positions in the Fc region, which are selected from the group consisting of:
(1)L309W+E345R;
(2)L309Y+E345R;
(3)L309E+E345R;
(4)L309F+E345R;
(5)L309H+E345R;
(6)L309C+E345R;
(7)L309D+E345R;
(8)L309N+E345R;
(9)L309Q+E345R;
(10)L309R+E345R;
(11)L309S+E345R;
(12)L309T+E345R; and
(13)L309K+E345R.

4. The antibody or fragment or fusion protein thereof of any one of claims 1-3, wherein the binding functional domain targeting the predetermined antigen binds to an antigen molecule selected from the group consisting of: CD38, CD3, CD47, CD19, CD20, HER2, EGFR, CD123, Glypican-3, CD25, Trop-2, EpCAM, and a combination thereof.

5. The antibody or fragment or fusion protein thereof of any one of claims 1-4, wherein the heavy chain Fc region element is derived from IgG, and the IgG has an amino acid sequence selected from the group consisting of:
SEQ ID NO: 31, SEQ ID NO: 32 and SEQ ID NO: 34.

6. The antibody or fragment or fusion protein thereof of any one of claims 1-5, wherein the antibody or fragment or fusion protein thereof is selected from the group consisting of:
(a) an antibody or fragment or fusion protein thereof having amino acid sequences selected from the group consisting of:
a heavy chain comprising a VH region containing H-CDR1 as shown in SEQ ID NO: 35, H-CDR2 as shown in SEQ ID NO: 36, and H-CDR3 as shown in SEQ ID NO: 37, and a heavy chain constant region with a W or Y mutation at position 309 of human IgG1 according to the EU numbering; and a light chain comprising a VL region containing L-CDR1 as shown in SEQ ID NO: 38, L-CDR2 as shown in SEQ ID NO: 39, and L-CDR3 as shown in SEQ ID NO: 40; or
a heavy chain comprising a VH region containing H-CDR1 as shown in SEQ ID NO: 11, H-CDR2 as shown in SEQ ID NO: 12, and H-CDR3 as shown in SEQ ID NO: 13, and a heavy chain constant region with a W or Y mutation at position 309 of human IgG1 according to the EU numbering; and a light chain comprising a VL region containing L-CDR1 as shown in SEQ ID NO: 14, L-CDR2 as shown in SEQ ID NO: 15, and L-CDR3 as shown in SEQ ID NO: 16; or
a heavy chain, comprising a VH region containing H-CDR1 as shown in SEQ ID NO: 35, H-CDR2 as shown in SEQ ID NO: 36, and H-CDR3 as shown in SEQ ID NO: 37, and a heavy chain constant region with a W or Y mutation at position 309 and a R mutation at position 345 of human IgG1 according to the EU numbering; and a the light chain comprising a VL region containing L-CDR1 as shown in SEQ ID NO: 38, L-CDR2 as shown in SEQ ID NO: 39, and L-CDR3 as shown in SEQ ID NO: 40; or
a heavy chain comprising a VH region containing H-CDR1 as shown in SEQ ID NO: 11, H-CDR2 as shown in SEQ ID NO: 12, and H-CDR3 as shown in SEQ ID NO: 13, and a heavy chain constant region with a W or Y mutation at position 309 and a R mutation at position 345 of human IgG1 according to the EU numbering; and a light chain comprising a VL region containing L-CDR1 as shown in SEQ ID NO: 14, L-CDR2 as shown in SEQ ID NO: 15, and L-CDR3 as shown in SEQ ID NO: 16; or
a heavy chain comprising a VH region containing H-CDR1 as shown in SEQ ID NO: 35, H-CDR2 as shown in SEQ ID NO: 36, and H-CDR3 as shown in SEQ ID NO: 37, and a heavy chain constant region as shown in SEQ ID NO: 31; and a light chain comprising a VL region containing L-CDR1 as shown in SEQ ID NO: 38, L-CDR2 as shown in SEQ ID NO: 39, and L-CDR3 as shown in SEQ ID NO: 40; or
a heavy chain comprising a VH region containing H-CDR1 as shown in SEQ ID NO: 11, H-CDR2 as shown in SEQ ID NO: 12, and H-CDR3 as shown in SEQ ID NO: 13, and a heavy chain constant region as shown in SEQ ID NO: 31; and a light chain comprising a VL region containing L-CDR1 as shown in SEQ ID NO: 14, L-CDR2 as shown in SEQ ID NO: 15, and L-CDR3 as shown in SEQ ID NO: 16; or
(b) a polypeptide derived from (a), that is formed by substitution, deletion, or addition of one or more amino acid residues to the amino acid sequence in (a), and has antigen binding function and CDC activity.

7. A method for improving the CDC of an antibody or fragment or fusion protein thereof, wherein the antibody or fragment or fusion protein thereof comprises a Fc region of immunoglobulin and a binding functional domain targeting a predetermined antigen, and the method comprises:
(S1a) Mutations in one or more amino acid residues are introduced into the antibody or fragment or fusion protein thereof, the mutation comprising that the amino acid at position 309 in the Fc region is mutated to an amino acid selected from the group consisting of: W, Y, E, F, H, C, D, N, Q, R, S, T, and K; and/or
(S1b) A tail-piece element is fused at the C-terminus of the Fc region, and the sequence of the tail-piece element is shown in SEQ ID NO: 7 or 8.

8. The method of claim 7, in step (S1a), the mutation further comprises that the position 345 in the Fc region of the IgG heavy chain is mutated to R.

9. A heavy chain Fc region element comprising:
(1) an amino acid residue at position 309 in the Fc region selected from the group consisting of: W, Y, E, F, H, C, D, N, Q, R, S, T, and K; preferably, further comprising that the position 345 in the Fc region of the IgG heavy chain is mutaed to R; and/or
(2) a tail-piece element at the C-terminus.

10. An isolated nucleic acid molecule encodeing the antibody or fragment or fusion protein thereof of any of claims 1-6, or the heavy chain Fc region element of claim 9.

11. An expression vector comprising the nucleic acid molecule of claim 10.

12. A host cell comprising the expression vector of claim 11.

13. A method for the preparation of the antibody or fragment or fusion protein thereof of any of claims 1-6, or the heavy chain Fc region element of claim 9, which comprises the following steps of:
(a) Under expression conditions, culturing the host cell of claim 12, thereby expressing the antibody or fragment or fusion protein thereof or the heavy chain Fc region element;
(b) Separating and purifying the antibody or fragment or fusion protein thereof or the heavy chain Fc region element as described in (a).

14. An immunoconjugate comprising:
(a) the antibody or fragment or fusion protein thereof of any one of claims 1-6; and
(b) a coupling moiety selected from the group consisting of a detectable label, a drug, a toxin, a cytokine, a radionuclide, or an enzyme.

15. A pharmaceutical composition comprising the antibody or fragment or fusion protein thereof of any one of claims 1-6 or the immunoconjugate of claim 14, and a pharmaceutically acceptable carrier.

16. Use of the antibody or fragment or fusion protein thereof of any one of claims 1-6, the immunoconjugate of claim 14, or the pharmaceutical composition of claim 15 in the preparation of a drug for the treatment of cancer or immune-related diseases.

17. The use of claim 16, wherein the cancer is selected from the group consisting of: melanoma, renal cancer, prostate cancer, pancreatic cancer, breast cancer, colon cancer, lung cancer, esophageal cancer, head and neck squamous cell cancer, liver cancer, ovarian cancer, cervical cancer, thyroid cancer, glioblastoma, glioma, multiple myeloma and other vegetative malignant diseases.

18. The use of claim 16, wherein the immune-related disease is an autoimmune disease, preferably autoimmune nephropathy (immune nephritis, autoimmune kidney disease), lupus, systemic lupus erythematosus (SLE), Sjogren's syndrome, arthritis, rheumatoid arthritis, asthma, COPD, pelvic inflammatory disease, Alzheimer's disease, inflammatory bowel disease, Crohn's disease, ulcerative colitis, Peyronie's disease, chylous diarrhea, gallbladder disease, hair hiding disease, peritonitis, psoriasis, psoriasis arthritis, vasculitis, surgical adhesion, stroke, type I diabetes, Lyme disease, meningoencephalitis, autoimmune uveitis, multiple sclerosis, Guillain Barr syndrome, atopic dermatitis, autoimmune hepatitis, ankylosing spondylitis, fibrotic alveolitis, Grave's disease, idiopathic thrombocytopenic purpura (ITP), Meniere's disease, pemphigus, primary biliary cirrhosis, sarcoidosis, scleroderma, Wegener's granulomatosis, other autoimmune disorders, pancreatitis, trauma (surgery), graft-versus-host disease, transplant rejection, heart diseases (including ischemic diseases such as myocardial infarction and atherosclerosis), intravascular coagulation, bone resorption, osteoporosis, osteoarthritis, periodontitis and hypochloremia, infertility related to lack of fetal maternal tolerance, vitiligo, myasthenia gravis (MG), systemic sclerosis, inflammatory bowel disease, gastritis or IgG4 related diseases, preferably immunoglobulin A nephropathy (IgAN), membranous nephropathy (MN), or monoclonal gammopathy of renal significance (MGRS), lupus nephritis, or purpura nephritis.
